# EUROPÄISCHE PATENTSCHRIFT

(11) **EP 0 530 794 B1**
(45) Veröffentlichungstag und Bekanntmachung des Hinweises auf die Patenterteilung: **12.04.2000**
(21) Anmeldenummer: 92115065.2
(22) Anmeldetag: 03.09.1992
(51) Int. Cl.: C12Q 1/68

(54) **Verfahren zum Nachweis ähnlicher Nukleinsäuren**
Method for detecting nucleic acids which are similar to one another
Méthode pour la détection d'acides nucléiques ayant une séquence similaire

(30) Priorität: 06.09.1991 DE 4129653
(43) Veröffentlichungstag der Anmeldung: 10.03.1993
(73) Patentinhaber: Roche Diagnostics GmbH, 68298 Mannheim (DE)
(72) Erfinder: Rust, Stephan, W-4400 Münster (DE); Funke, Harald, Dr., W-4400 Münster (DE); Assmann, Gerd, Prof. Dr., W-4400 Münster (DE); Rüger, Rüdiger, Dr., W-8124 Seeshaupt (DE); Kessler, Christoph, Dr., W-8021 Dorfen (DE)

(56) Entgegenhaltungen:
- EP-A- 0 333 465
- EP-A- 0 422 762
- WO-A-89/10414
- WO-A-90/11372
- NUCLEIC ACIDS RESEARCH. Bd. 17, Nr. 7, 1989, ARLINGTON, VIRGINIA US Seiten 2503 - 2516 C.R.NEWTON ET AL. 'Analysis of any point mutation. The amplification refractory mutation system (ARMS)'

## Beschreibung

Gegenstand der Erfindung sind ein Verfahren zum Nachweis ähnlicher Nukleinsäuren, ein Satz von Oligonukleotiden, die dafür geeignet sind, ein Reagenzkit, mit dem das erfindungsgemäße Verfahren durchgeführt werden kann und diverse Verwendungsmöglichkeiten des Verfahrens in der Diagnostik von Genen und Infektionen.

Die Untersuchung von Proben auf Anwesenheit bestimmter Nukleinsäuren oder Nukleinsäuregruppen erhält in jüngerer Zeit immer größere Bedeutung. Dies liegt zumindest zum Teil darin begründet, daß die Nukleotidsequenzen der Nukleinsäuren für Organismen ein charakteristisches Merkmal darstellen. In jüngster Zeit wird sogar der Versuch unternommen, Unterschiede in nur einem einzigen Nukleotid der Sequenz zur Unterscheidung von Nukleinsäuren auszunutzen. Solche Unterschiede können z.B. durch Punktmutationen entstandene Nukleotidaustausche sein. Natürliche Beispiele solcher nahe verwandter Nukleinsäuren sind die Allele, d.h. alternative Sequenzvarianten eines Gens an einem definierten Ort im Chromosom.

Aus Oncogene Research 1, 235-241 (1989) und Nucl. Acids Res. 17, 8093-8099 (1989) ist ein Verfahren bekannt, bei dem der gegebenenfalls die allele Variante enthaltende Bereich der Nukleinsäure zunächst durch Polymerase Chain Reaction (PCR) unter Verwendung von Primern mit speziellem Design amplifiziert und anschließend mit einem Restriktionsenzym behandelt wird. Auf diese Weise ist eine Diagnose der Allele durch eine Restriktionsfragmentpolymorphismus (RFLP)-Analyse möglich. Die elektrophoretische Auftrennung der Spaltprodukte nach ihrer Länge gibt dann Aufschluß darüber, ob das entsprechende Allel in der Probe enthalten war oder nicht. Dieses Verfahren hat den Nachteil, daß ein spezifischer Restriktionsverdau vorgenommen werden muß. Abgesehen davon, daß dies aufwendig ist, muß für jede mögliche Mutation, die nicht schon per se einen RFLP liefert, auch ein Design für einen der Punktmutation benachbarten Primer möglich sein, welches den Verdau mit einem Restriktionsenzym erlaubt, das genau an der besagten Stelle schneidet. Dies kann auf die dort beschriebenen technischen Schwierigkeiten stoßen.

In EP-A-0 332 435 ist ein Verfahren beschrieben, mit dem prinzipiell eine Nukleinsäure selektiv neben einer anderen Nukleinsäure nachgewiesen werden kann, die sich nur in einem Nukleotid von ihr unterscheidet. Dazu wird der Effekt ausgenutzt, daß theoretisch nur solche an die nachzuweisende Nukleinsäure hybridisierten Oligonukleotide enzymatisch verlängert werden können, deren in Verlängerungsrichtung endständiges Nukleotid komplementär zu dem entsprechenden Nukleotid der nachzuweisenden Nukleinsäure (des einen Allels) ist. Das Oligonukleotid wird daher so gewählt, daß nur mit der nachzuweisenden Nukleinsäure eine solche Komplementarität besteht. Das an das andere Allel anhybridisierte Oligonukleotid wird so theoretisch nicht verlängert. Es hat sich herausgestellt, daß in der Praxis in geringem Umfang jedoch auch eine Verlängerung des an das andere Allel hybridsierten Oligonukleotids vorkommt, was die Empfindlichkeit und vor allem die Spezifität des Nachweises der zu untersuchenden Nukleinsäure herabsetzt. Unspezifische Verlängerungen treten vor allem dann leicht auf, wenn am Mismatch am 3'-Ende ein T beteiligt ist oder es sich um ein C:A Mismatch handelt (Kwok et al., Nucleic Acids Research 18, 999-1005 (1990) ). In EP-A-0 332 435 wurde zur Steigerung der Spezifität vorgeschlagen, die Nukleotidsequenz des Oligonukleotids so zu wählen, daß im Endbereich ein weiteres Nukleotid nicht zu dem entsprechenden Nukleotid der beiden Nukleinsäuren komplementär ist. Zum Nachweis beider Allele müssen 2 Reaktionen durchgeführt werden, wobei pro Reaktion nur die Präsenz eines der Allele nachgewiesen werden kann. Dazu werden zwei allelspezifische Primer und ein Gegenstrangprimer synthetisiert, und in 2 Reaktionen wird die Probe einmal in einer PCR mit dem Gegenstrangprimer und einem der allelspezifischen Primer amplifiziert und in einer zweiten, parallelen Reaktion in einer PCR mit dem Gegenstrangprimer und dem anderen allelspezifischen Primer amplifiziert. Wenn in einer der Reaktionen das betreffende allelspezifische PCR-Produkt nicht gefunden wird, wird angenommen, daß das betreffende Allel in der untersuchten Probe nicht vorhanden ist. Da homozygote DNA-Proben nur eines der beiden Allele enthalten, welches dann jeweils nur in der einen der beiden Reaktionen nachzuweisen ist, müssen zur Kontrolle der Effizienz der jeweils für das andere Allel spezifischen PCR-Reaktion und zum Beweis der Abwesenheit des betreffenden Allels zwei weitere Primer eingesetzt werden, die in allen Reaktionen das gleiche, vom spezifischen Produkt unterscheidbare Kontrollprodukt liefern. Liegt in einem PCR-Produkt das Kontrollprodukt vor, wird aber kein spezifisches Produkt des untersuchten Allels gefunden, so wird angenommen, daß die untersuchte Probe das in der angesetzten Reaktion nachzuweisende Allel nicht enthält. Bei dieser Methodik müssen also zwei Allele durch zwei getrennte Reaktionen nachgewiesen bzw. ausgeschlossen werden und in jede einzelne PCR-Reaktion muß eine Kontroll PCR eingeschlossen werden.

Eine Förderung der Selektivität durch Absenkung der dNTP Konzentration wurde in Biochem. Biophys. Res. Commun. 160, 441-447 (1989) vorgeschlagen. Selbst wenn diese Zusatzmaßnahme getroffen wurde, kann es aber bei dem Versuch, die Allele in getrennten Reaktionsansätzen nachzuweisen, zu unspezifischen Produkten kommen.

In der Ligase chain reaction (LCR; WO 89/09835) wird eine thermostabile Ligase eingesetzt, um spezifisch zwei benachbarte Oligonukleotide zu verknüpfen, und zwar nur dann, wenn sie bei stringenter Hybridisierungstemperatur an ein komplementäres Target hybridisiert sind und eine vollständige Basenpaarung an der Verknüpfungsstelle vorliegt. Unterscheiden sich zwei Allele durch eine Mutation an der Position der Verknüpfungsstelle, so ist die genannte Bedingung der vollständigen Basenpaarung nur für eines der Allele erfüllt. Zwei weitere Oligonukleotide, komplementär zu den ersten beiden, werden zusätzlich gebraucht, um in der Ligase chain reaction das Ligationsprodukt zu amplifizieren. Zum Nachweis von zwei Allelen wurden bisher 2 Reaktionen mit mindestens insgesamt 6 Oligonukleotiden durchgeführt, der Nachweis des Amplifikations-Produkts erfolgte durch eine radioaktive Markierung (Proc. Natl. Acad. Sci. USA 88, 189-193 (1991) ).

Aus Proc. Natl. Acad. Sci. USA 1985, Vol. 82, 1585-1588 und New England Journal of Medicine 317, 985 (1987) ist ein Allelnachweis bekannt, der auf der differentiellen Hybridisierung von "allelspezifischen" Oligonukleotiden (ASO) mit den Allelen beruht. Hierzu werden 2 Oligonukleotide von z.B. je 20 Basen Länge synthetisiert, jeweils eines passend zu einem der beiden unterschiedlichen Allele, aber mit einem etwa in der Mitte der Oligonukleotidsequenz gelegenen Mismatch zum jeweils anderen Allel. Durch differentielle Hybridisation mit markierten Oligonukleotiden ist so eine Unterscheidung von Allelen sowohl bei Analyse von humaner genomischer DNA, als auch bei Analyse von PCR-Produkten möglich.

Mit diesem Verfahren ist zwar auch eine direkte und diskrete Analyse genomischer DNA möglich, daß heißt ohne PCR, es sind dann aber zusätzliche Verdauungs- und Elektrophoreseschritte nötig.

In Nucleic Acids Research 17, 2437-2448 (1989) bzw. EP-A-0 333 465 wird eine Methode beschrieben, die es erlaubt, voramplifizierte humane genomische DNA durch Kompetition von allelselektiven Primern in wenigen weiteren PCR-Zyklen auf die Präsenz verschiedener Allele zu untersuchen (competitive oligonucleotide priming = COP). Dabei wird die oben beschriebene ASO-Technik in eine PCR-Technik übertragen. Bei der ursprünglichen ASO-Technik ist ein Fehler von z.B. 5% durch Kreuzhybridisation noch tolerierbar, da dann durch Intensitätsvergleiche der Signale bei entsprechenden Kontrollen eine absolut eindeutige Interpretation der Ergebnisse möglich ist. Bei einer PCR-Reaktion, in der allelspezifische Oligonukleotide als Primer eingesetzt werden, würde ein derartiger Fehler bei einem Reaktionsansatz, in dem die Amplifikation beider Allele möglich ist aber nach 10 Zyklen bereits zu einem Fehler von ca. 12% führen, wenn eine Probe nur eines der Allele enthält. Zwar konnten Gibbs et al. zeigen, daß die Kompetition der Primer die Selektivität erhöht, dennoch wurde der interessierende Bereich der genomischen DNA zunächst durch PCR amplifiziert und dann die Analyse für die verschiedenen Allele in 10 weiteren Zyklen durchgeführt, wobei zwei allelselektive Primer und ein Gegenstrangprimer in diesen 10 Zyklen eingesetzt wurden, und für zwei durchzuführende Reaktionen jeweils einer der allelselektiven Primer radioaktiv markiert wurde. Ein selektiver Nachweis von verschiedenen Allelen konnte für Oligonukleotide mit einer Länge von 12 bis 16 Basen gezeigt werden, bei längeren Oligonukleotiden wurde unter den beschriebenen Bedingungen jedoch auch unspezifisches Produkt erhalten.

Die auf differentieller Hybridisierung beruhenden Verfahren sind bisher beschränkt auf ganz bestimmte Situationen und überdies sehr aufwendig in Bezug auf die Durchführung und störanfällig. Darüber hinaus ist die Voramplifikation bei der COP ein Verfahrensschritt, den man gerne einsparen würde.

In WO 90/11372 ist ein Verfahren zur Amplifizierung und zum Nachweis von mehreren Nukleinsäuren beschrieben, bei dem zweiteilige Primer eingesetzt werden. Die ersten Teile sind komplementär zu den unterschiedlichen nachzuweisenden Allelen und die zweiten Teile unterscheiden sich durch jeweils einen einzigartigen tail.

In Nucleic Acids Research, Vol. 17, 7, 2503 ― 2516 (1989) ist die Verwendung von 3'-Mismatchprimern zur Differenzierung zwischen Nukleinsäuren beschrieben. Es ist ferner offenbart, daß die Einführung weiterer Mismatches der Oligonukleotide zu den nachzuweisenden Nukleinsäuren, insbesondere in der Nähe des 3'-Endes, die Primer/Templatkomplexe weiter destabilisiert.

Aufgabe der Erfindung war es, ein einfaches Verfahren zum spezifischen Nachweis von ähnlichen Nukleinsäuren bereitzustellen, welches keinen Restriktionsverdau benötigt, und bei dem es möglich ist, die ähnlichen Nukleinsäuren in einem Reaktionsansatz zu amplifizieren.

Gegenstand der Erfindung ist daher ein Verfahren zum Nachweis ähnlicher Nukleinsäuren, deren Nukleotidsequenzen sich in mindestens einer Position (X) unterscheiden, welches mindestens folgende Teilschritte enthält:
a) Umsetzung eines Satzes von Oligonukleotiden, deren Nukleotidsequenzen sich in der der Position X entsprechenden Position unterscheiden, mit den nachzuweisenden Nukleinsäuren unter Hybridisierungsbedingungen:
b) Verlängerung der anhybridisierten Oligonukleotide unter Verwendung der nachzuweisenden Nukleinsäuren als Template in einem einzigen Reaktionsgefäß; und
c) Nachweis von Verlängerungsprodukten
   - wobei sich die Oligonukleotide des Satzes außerdem in mindestens einer weiteren Position (Y) voneinander unterscheiden, wobei die Positionen X und Y im hybridisierenden Bereich liegen, so daß sich die Verlängerungsprodukte aus b) in mehr als einer Position im hybridisierenden Bereich voneinander unterscheiden und
   - in Schritt c) die in Schritt b) erzeugten Unterschiede der Verlängerungsprodukte zu den ähnlichen Nukleinsäuren sowie die Position (X) zum Nachweis der Verlängerungsprodukte verwendet werden.

Ähnliche oder nahe verwandte Nukleinsäuren im Sinne der Erfindung sind Nukleinsäuren, deren Nukleotidsequenzen im wesentlichen identisch sind, die jedoch an mindestens einer Position der Nukleotidsequenz einen Unterschied aufweisen. Diese Position wird im folgenden mit X bezeichnet. Wenn mehrere Unterschiede zum Gegenstand der Erfindung gehören, werden diese mit X1, X2...XN bezeichnet. Solche Unterschiede kommen beispielsweise durch Punktmutationen (z.B. Substitutionen durch andere Basen oder auch durch Deletionen oder Insertionen einer oder mehrerer Basen zustande. Die einander ähnlichen Nukleinsäuren werden üblicherweise dann als Allele bezeichnet.

Bedeutsame Punktmutationen findet man beispielsweise im LDL-Rezeptorgen (Arteriosklerosis 9, 1-8 bis 1-13 (1989)) oder im Apolipoprotein-B-Gen (CGG―>CAG-Mutation des Codons 3500; Proc. Natl. Acad. of Sciences USA 86, S. 587-591 (1989)), im Gen der viruseigenen reversen Transkriptase von HIV (A―>T-Mutation im Codon 215; Science 246, S. 1155-1158 (1989)) bzw. im β-Globin-Gen der A --->T Austausch im Codon 6 bei Sichelzellanämie. Außerdem finden sich ähnliche Nukleinsäuren in vielen nahe verwandten Organismen, beispielsweise Bakterien und Viren (Serovarianten). Insbesondere ähneln sich Nukleinsäuren einzelner Bakterienstämme in der Sequenz ihrer ribosomalen Gene bzw. der rRNA sehr stark. Ähnliche Nukleinsäuren im Sinne der Erfindung sind jedoch auch Kontrollnukleinsäuren, die einer nachzuweisenden Nukleinsäure ähneln. Die Position X wird auch als polymorpher Ort bezeichnet. Die nachzuweisenden Nukleinsäuren können RNA oder DNA sein. Bei Bakteriendiagnostik hat sich rRNA als besonders zweckmäßig erwiesen.

Als Original-Target wird die Nukleinsäure bezeichnet, die in der Probe enthalten war. Die später mit ihrer Verwendung entstehenden Polynukleotide, die ebenfalls wieder als target-Nukleinsäure verwendet werden und die meist kürzer sind als das Originaltarget, werden als Produkttarget bezeichnet.

Wenn im Folgenden vom Nachweis zweier Nukleinsäuren bzw. Allele die Rede ist, so können diese Ausführungsformen in einfacher Weise auf den Nachweis von Nukleinsäuren mit allen denkbaren Unterschieden in X (z.B. aller denkbaren Allele) durch Anwendung entsprechender Oligonukleotide übertragen werden.

Als Position wird eine definierte Stelle auf einer Nukleinsäure bezeichnet. Diese Position kann beispielsweise von einem Nukleotid belegt sein.

Als komplementär werden Nukleotide bezeichnet, welche eine reguläre, Watson-Crick-Basenpaarung gewährleisten (z.B. G-C, A-T bzw. A-U). Diese führen zu sogenannten Matches, nicht-komplementäre zu Mismatches.

Sofern andere Basen oder Basenanaloge eine solche Basenpaarung zulassen (7-deaza-dGTP, dITP) so werden auch sie als komplementär bezeichnet.

Zur Durchführung des erfindungsgemäßen Nachweisverfahrens müssen die nachzuweisenden Nukleinsäuren in für in-vitro-Reaktionen geeigneter Form vorliegen. Dazu wird eine zu untersuchende Probe aus beispielsweise einem Gewebe, einzelnen Zellen oder einer zellhaltigen Flüssigkeit zunächst auf bekannte Weise aufgeschlossen, um die Zellwände aufzubrechen (z.B. thermische, enzymatische oder chemische Lyse bzw. Kombinationen davon).

Falls die Nukleinsäuren nicht in einzelsträngiger Form vorliegen, werden sie in solche auf bekannte Weise überführt (z.B. thermische oder alkalische Denaturierung, enzymatische Strangtrennung oder Destabilisierung der Doppelstränge unter bestimmten Salzbedingungen).

Anschließend wird die Probe mit einem Satz von Oligonukleotiden in Kontakt gebracht. Dabei werden Bedingungen gewählt, bei denen die erfindungsgemäßen Oligonukleotide mit den zugehörigen Bereichen der nachzuweisenden Nukleinsäuren hybridisieren. Diese Hybridisierung wird allgemein auch als Annealing bezeichnet. Eine Hybridisierung mit nicht-nachzuweisenden Nukleinsäuren kann durch Wahl der Nukleotidsequenz, Länge, Temperatur, Hilfsstoffe etc. vermieden werden.

Der erfindungsgemäße Satz von Oligonukleotiden besteht aus mindestens so vielen verschiedenen Oligonukleotiden, wie verschiedene Nukleinsäuren nachgewiesen werden sollen. Beim Nachweis zweier verschiedener Nukleinsäuren sind daher mindestens zwei verschiedene Oligonukleotide (und ggf. Gegenstrangoligonukleotide) erforderlich. Die eingesetzten Oligonukleotide haben bevorzugt eine Länge von weniger als 100, besonders bevorzugt von 5 bis 50 nt. Jedes Oligonukleotid hat eine Nukleotidsequenz, die zu dem in Nachbarschaft des Nukleotids X liegenden Bereich so stark komplementär ist, daß das Oligonukleotid mit der nachzuweisenden Nukleinsäuren hybridisieren kann. Die erfindungsgemäßen Oligonukleotide eines Satzes unterscheiden sich jedoch in ihrer Sequenz auf ganz bestimmte Weise.

Ein Unterschied in der Nukleotidsequenz der Oligonukleotide eines Satzes befindet sich in der Position, welches im anhybridisierten Zustand der Position X der Nukleinsäure entspricht. Dieser Unterschied kann durch Substitution mit einer anderen Base oder Basenanalogen oder Deletion/Insertion zustandekommen.

Im Sinne der Erfindung wird das Oligonukleotid als "match"-Oligonukleotid bezeichnet, welches an Position X ein Nukleotid aufweist, welches zu dem Nukleotid der Position X der nachzuweisenden Nukleinsäure komplementär ist. Die zu dem match-Oligonukleotid passende Nukleinsäure (mit der eine Verlängerung stattfindet), wird als target-Nukleinsäure bezeichnet. Als "mismatch"-Oligonukleotid wird ein Oligonukleotid bezeichnet, dessen Nukleotid in Position X ein Nukleotid ist, das nicht zu dem Nukleotid in Position X der zur Betrachtung stehenden nachzuweisenden Nukleinsäure komplementär ist.

Je nach gewählter Variante des erfindungsgemäßen Verfahrens liegt das Nukleotid X an einer bestimmten Stelle der Oligonukleotide, beispielsweise in der Mitte oder an einem Ende der Oligonukleotide. Im ersten Fall ist das erfindungsgemäße Verfahren eine Verbesserung des Verfahrens welches auf kompetitivem Priming beruht (im Folgenden Variante 1 genannt) und im zweiten Fall des Mismatch-Priming-Verfahrens der EP-A-0 332 435 und verwandter Verfahren (im Folgenden Variante 2 genannt). In jedem Fall jedoch wird für jede der nachzuweisenden Nukleinsäuren ein Oligonukleotid eingesetzt, dessen dem Nukleotid in Position X der nachzuweisenden Nukleinsäure entsprechendes Nukleotid zu diesem Nukleotid komplementär ist. Dies bedeutet, daß dieses Oligonukleotid in Position X ein Nukleotid enthält, welches zu den entsprechenden Nukleotiden der übrigen in der Probe vorhandenen nachzuweisenden in Bezug auf Position X unterschiedlichen Nukleinsäuren nicht komplementär ist.

Die erfindungsgemäßen Oligonukleotide unterscheiden sich in mindestens einer weiteren Position Y im hybridisierenden Bereich voneinander. Dies kann erreicht werden durch Substitutionen duch andere Basen oder Basenanalogen und/oder Deletionen/Insertionen in der Sequenz des Oligonukleotids. Mindestens eines, bevorzugt jedoch jedes der erfindungsgemäßen Oligonukleotide eines Satzes weist an einer Nukleotidposition Y bevorzugt ein Nukleotid auf, welches zu dem entsprechenden Nukleotid der nachzuweisenden Nukleinsäuren nicht komplementär ist. Wesentlich für die optimale Durchführung des erfindungsgemäßen Verfahrens ist, daß für die Oligonukleotide eines Satzes sich diese Positionen Y in unterschiedlicher relativer Position zu X befinden. Für Variante 1 ist die Lage von Y auf den Oligonukleotiden nicht kritisch. Sie kann daher im wesentlichen frei gewählt werden.

Position Y befindet sich bei Variante 2 jeweils bevorzugt in der Nähe der Nukleotidposition X im 3'-Endbereich der Oligonukleotide. Bevorzugt liegt die Position Y 1-8, bevorzugt 1-3 Nukleotide von X entfernt. Beispielsweise hat es sich als günstig erwiesen, wenn Y des einen Oligonukleotids, das auf das Endnukleotid (Position X) folgende Nukleotid und Y des anderen Oligonukleotids das zwei oder drei Nukleotide vom Ende entfernte Nukleotid ist. In einem weiteren Oligonukleotid (zum Nachweis von drei ähnlichen Nukleinsäuren) könnte dann beispielsweise das nicht komplementäre Nukleotid drei oder zwei Nukleotide vom Endnukleotid entfernt sein, usw.

Im Folgenden werden die Positionen Y der 1, 2 ... N Oligonukleotide eines Satzes mit Y1, Y2...YN bezeichnet. Jedes Oligonukleotid kann auch an mehreren Positionen Nukleotide aufweisen, die der Definition des Nukleotids an Position Y entsprechen. Bevorzugt enthält das Oligonukleotid 1-5 solche Nukleotide.

Zum Einsatz gelangt daher bevorzugt ein Satz von Oligonukleotiden, welcher mindestens zwei erfindungsgemäße Oligonukleotide enthält, wobei sich diese mindestens in ihren X entsprechenden Nukleotiden und jeweils zwei weiteren Nukleotiden unterscheiden. Diese Anordnung von nicht komplementären Nukleotiden in den Oligonukleotiden hat zur Folge, daß in keiner Phase der anschließend durchgeführten Verlängerungsreaktion, wie beispielsweise einer PCR gemäß EP-A-0 200 362, ein Verlängerungsprodukt entsteht, welches mit einem anderen Oligonukleotid des Satzes nur noch einen Unterschied in dem Nukleotid in Position X aufweist. Das Aufrechterhalten einer zweiten unterschiedlichen Nukleotidposition ist jedoch für die Empfindlichkeit des Nachweises sehr wichtig, wie aus der EP-A-0 332 435 bekannt ist. Oligonukleotiden eines Satzes ist mindestens ein Nukleotidsequenzbereich gemeinsam, der außer in Position Y zu einer gemeinsamen Sequenz der nachzuweisenden Nukleinsäuren komplementär ist. Diese Bereiche umfassen bevorzugt mindestens 50% der Länge der Oligonukleotide.

Nach der Hybridisierung der Oligonukleotide an die nachzuweisenden Nukleinsäuren werden die entstandenen Hybride einer Verlängerungsreaktion unterzogen. Eine solche Verlängerungsreaktion besteht bevorzugt im Anhängen von Mononukleotiden, welche zu den entsprechenden Nukleotiden der Originaltarget-Nukleinsäure komplementär sind, an das Oligonukleotid in 3'----5'-Richtung. Dies geschieht bevorzugt dann wenn das in Verlängerungsrichtung endständige Nukleotid ds anhybridisierten Oligonukleotids zu dem entsprechenden Nukleotid der einen Nukleinsäure komplementär ist (match) und von der chemischen Struktur her verlängerbar ist. Die Reaktionsbedingungen für eine solche Kettenverlängerung mittels einer Polymerase und Mononukleotiden sind beispielsweise aus der EP-A-0 201 184 oder EP-A-0 332 435 bekannt.

Außerdem kann die Verlängerung durch Anknüpfen eines weiteren Oligonukleotids an das bereits anhybridisierte match-Oligonukleotid bewerkstelligt werden, wobei das anzuhängende Oligonukleotid im wesentlichen komplementär sein muß zu dem einzelsträngigen Bereich der Nukleinsäure, der sich an den Bereich anschließt, an den das match-Oligonukleotid bereits anhybridisiert ist. Diese Art der Verlängerung kann mit einer Ligasereaktion erreicht werden. Eine solche Verlängerungsreaktion ist beispielsweise in Proceedings of the Natl. Acad. of Sciences USA 88, S. 189-193, (1991) oder WO 89/09835 beschrieben. Weitere Amplifikationsverfahren auf welche die Erfindung angewendet werden kann, sind Repair Chain Reaction (WO 90/01069), das Verfahren der DE-A-4010465 und die erste Stufe des Verfahrens aus WO 91/03573.

Bei Variante 1 werden alle an nachzuweisenden Nukleinsäuren anhybridisierten Oligonukleotide verlängert. Da das Mismatch-Oligonukleotid P1 der einen Nukleinsäure jedoch mindestens zwei Mismatch-Nukleotide (X, Y1) aufweist, hybridisiert es unter kompetitiven Bedingungen nicht so gut mit dieser Nukleinsäure, wie das Match-Oligonukleotid P2, welches an der Position X das Matchnukleotid aufweist, wobei vorausgesetzt wird, daß die Y-Positionen in den beiden jeweils kompetitiven Oligonukleotiden so gewählt sind, daß die Kompetition der Oligonukleotide trotzdem von der jeweiligen Base an Position X entschieden wird (und nicht etwa durch die Unterschiede in den Y-Positionen). Die Oligonukleotide können bei Variante 1 auch gegeneinander versetzt auf dem Originaltarget hybridisiert sein, wobei jedoch die Positionen X und bevorzugt auch die Positionen Y in dem gemeinsamen Bereich liegen. Hauptprodukt der Verlängerungsreaktion ist daher eine Nukleinsäure, die auf der Verlängerung des Match-Oligonukleotids beruht.

Bei Variante 2 können zwar auch Mismatch-Oligonukleotide an die jeweils nachzuweisende Nukleinsäure hybridisieren, eine Verlängerung findet jedoch nur für das Match-Oligonukleotid statt.

Voraussetzung für die Spezifität der Verlängerungsreaktion bei Variante 2 ist, daß das Enzym, welches die Verlängerungsreaktion ausführt, nicht völlig unabhängig davon, ob Mismatches vorliegen, eine Verlängerung der Oligonukleotide katalysiert. Diese Voraussetzungen sind jedoch beispielsweise bei Thermus aquaticus-DNA-Polymerase (EP-A-0 258 017), oder E.coli-Ligase erfüllt.

Zur Amplifikation des Bereiches, welcher zwischen den äußeren Enden der Oligonukleotide liegt, die an die nachzuweisenden Nukleinsäuren hybridisiert werden, werden die endstandenen Verlängerungsprodukte beispielsweise mit Oligonukleotiden umgesetzt, die zu den Elongaten im wesentlichen komplementär sind (Gegenstrangoligonukleotide) und zur Hybridisierung gebracht. Diese Oligonukleotide werden unter Verwendung der im ersten Schritt hergestellten Verlängerungsprodukte als Template verlängert, wobei die Position X in der Regel in dem Bereich der Verlängerung liegt. Die Gegenstrangoligonukleotide können für alle nachzuweisenden Nukleinsäuren gleich sein. Sie können jedoch ebenfalls allelselektiv sein.

Auch diese Verlängerung kann durch Anhängen von Mononukleotiden oder Oligonukleotiden durchgeführt werden.

Bei Verwendung der erfindungsgemäßen Oligonukleotide, die sich außer in Position X noch in mindestens zwei weiteren Positionen unterscheiden, entsteht in der zweiten Verlängerungsreaktion (im folgenden Zyklus 2 genannt) ein Produkt, welches zu allen anderen Oligonukleotiden außer dem, aufgrund dessen Hybridisierung mit der nachzuweisenden Nukleinsäure das Produkt selbst entstanden ist (dem ursprünglichen match-Oligonukleotid), mindestens 3 (also mehr als ursprünglich vorhanden) nicht komplementäre Nukleotide aufweist. Damit ist eine Verlängerung von mismatch-Oligonukleotiden an diesem Produkt erschwert. In analoger Weise kann durch zusätzliche Y Positionen das fälschliche Annealing an das Amplifikationsprodukt des jeweils anderen Allels erschwert werden. Dadurch wird eine sonst mögliche Blockierung des Amplifikationsproduktes für das passende Oligonukleotid, und eine damit verbundene Absenkung der Ausbeute vermieden.

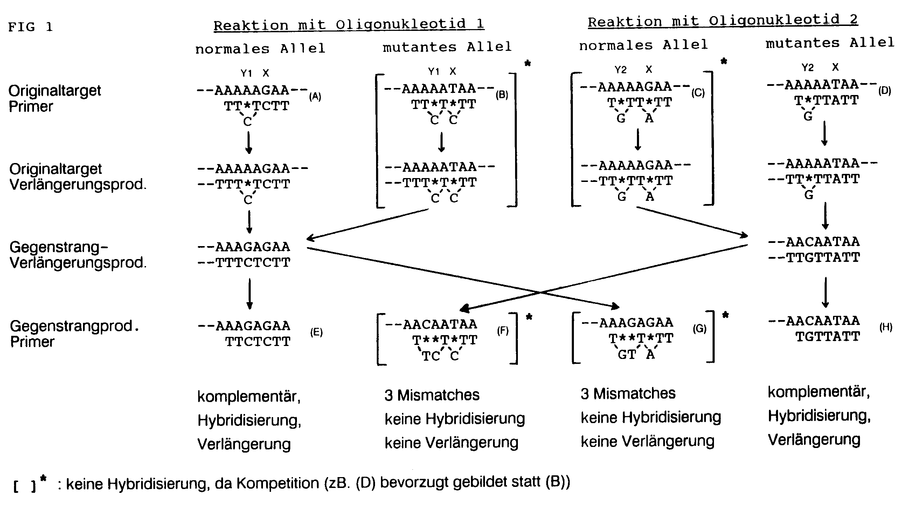

Diese Verbesserung der bekannten Verfahren wird im Folgenden unter Bezugnahme auf Fig. 1 bis 4 erläutert.

Hauptprodukt der Verlängerungsreaktion bei Variante 1 ist eine Nukleinsäure, die auf der Verlängerung des Match-Oligonukleotids beruht. Eine Hybridisierung des Mismatch-Oligonukleotids an ein Verlängerungsprodukt aus Zyklus 2 aus der Hybridisierung mit dem Match-Oligonukleotid ist praktisch unmöglich, da dann schon drei Mismatches (X, Y1, Y2) vorhanden sind. Diese Situation ist beispielhaft in Fig. 1 gezeigt. Praktisch wird der Erfolg insbesondere durch geschickte Wahl der Annealing-Temperatur (gewünschtenfalls auch anderer Reaktionsparameter, wie Konzentration der Oligonukleotide) erreicht.

Durch Erhitzen auf z.B. 90°C, Abkühlen auf eine Temperatur kurz oberhalb des T_{M}-Wertes beider Primer am Originaltarget und anschließend langsames Abkühlen auf eine Temperatur kurz oberhalb des T_{M}-Wertes beider Primer am Originaltarget und anschließend langsames Abkühlen auf eine Temperatur deutlich unterhalb der T_{M}-Werte beider Primer wird dann erreicht, daß die allelselektiven Primer jeweils an das besser passende Target hybridisieren. Der T_{M}-Wert ist definiert als die Temperatur, bei der 50% einer Targetsequenz mit einem Primer hybridisiert sind. Bei dieser Temperatur kann ein gebundenes passendes Oligonukleotid auch rasch wieder freigesetzt werden (Gleichgewicht). Für ein weniger gut passendes Oligonukleotid liegt der T_{M}-Wert am gleichen Target aber niedriger, d.h. bei diesem niedrigeren T_{M}-Wert wird das weniger passende Oligonukleotid kaum an das genannte Target binden, sondern von dem nun mehr als 50% bindenden passenden Oligonukleotid verdrängt. Dieses Annealing läuft bei den entsprechenden Temperaturen bei beiden Allelen ab. Bei der Amplifikation des PCR-Produkts sind die T_{M}-Werte für die jeweils perfekt komplementäre Target Kombination [(E), (H), Fig. 1] deutlich erhöht im Vergleich zu den T_{M}-Werten der Oligonukleotid/Target Kombinationen [(A), (B), (C), (D) Fig. 1] , da dort die Zusatzmutationen den jeweiligen T_{M}-Wert durch Mismatchbildung abgesenkt hatten. Dagegen ist für die Oligonukleotid/PCR-Produkttarget-Kombination, bei denen ein Oligonukleotid mit dem nicht passenden Allel hybridisiert [(F), (G), Fig. 1] der T_{M}-Wert nochmals deutlich niedriger als am Originaltarget, da nun die entsprechend der vorliegenden Erfindung gewählten Zusatzmutationen aus beiden Oligonukleotiden in einem solchen Paar akkumulieren. Dadurch ist also eine weitere Erhöhung der Spezifität der Amplifikation der PCR-Produkte erreicht. Wird nun zum Annealing in der PCR eine Temperatur gewählt, die knapp unterhalb der T_{M}-Werte der perfekt komplementären Kombinationen liegt, so ist das falsche Annealing an PCR-Produkten (F), (G) oder am Originaltemplate (B), (C) praktisch ausgeschlossen. Gegenüber dem bekannten COP-Verfahren hat die Variante 1 den Vorteil, daß eine Differenzierung durch Temperaturwahl in den Folgezyklen nur grob oder garnicht stattfinden muß. Außerdem wird der direkte Nachweis genomischer Nukleinsäuren ohne Voramplifikation möglich.

Die hier beschriebene Variante ist besonders geeignet für Multiplexanalysen, da in dem genannten, vor der PCR durchgeführten Annealingschritt nicht nur die T_{M}-Werte für eine Mutation und die entsprechenden Primer erreicht werden können, sondern, durch langsames Abkühlen über einen Bereich vom höchsten T_{M}-Wert bis unter den niedrigsten T_{M}-Wert, allen Oligonukleotiden ein selektives Annealing ermöglicht wird.

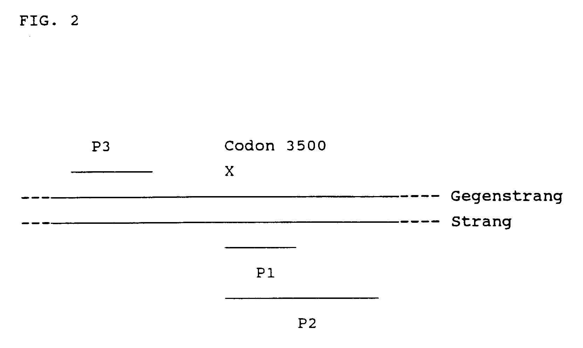

Die zweite Variante wird anhand der Figuren 2-4 erläutert. Dieser Fall, bei dem das 3'-Endnukleotid der Oligonukleotide der Position X entspricht, ist wegen seiner höheren Selektivität bevorzugt. Dies ist darin begründet, daß es für die Selektivität oft praktisch nicht darauf ankommt, ob das Mismatch-Oligonukleotid anhybridisiert ist oder nicht.

Zur Veranschaulichung des Prinzips sei z.B. ein PCR-Reaktionsansatz in einem einzigen Reaktionsgefäß betrachtet, in dem gleichzeitig zwei Allele eines Gens detektiert werden können, die sich in einer Basenposition unterscheiden (ein Allel habe dort die Base M(utant) das andere N(ormal)). Dabei werden drei Primer eingesetzt, ein Gegenstrangprimer und je ein Primer selektiv für je eines der Allele. Die Primer sind dadurch selektiv, daß die Base am 3'-Ende des einen selektiven Primers zu M, die des anderen Primers zu N komplementär ist. Die beiden allelselektiven Primer werden nun aber z.B. durch zwei zusätzliche Substitutionen voneinander unterschieden, wobei in jedem Primer je ein mismatch zum Template entsteht, so daß jeder der beiden Primer ein mismatch zum jeweils selektierten Allel hat und zwei mismatches zum anderen Allel. Zur einfachen Unterscheidung der resultierenden PCR-Produkte können außerdem (z.B.) verschieden lange allelselektive Primer gewählt werden. Diese Veränderungen werden in die jeweiligen PCR-Produkte eingebaut, so daß im weiteren Verlauf der PCR das durch jeweils einen der allelselektiven Primer entstandene PCR-Produkt ein besseres (nämlich vollständig komplementäres) Target für diesen Primer darstellt als das Originaltarget, und außerdem für den jeweils anderen allelselektiven Primer kein geeignetes Target mehr darstellt, da dieser nun sogar drei mismatches zu dem veränderten Target hat. Dadurch liegt die fehlerbestimmende Selektion der Allele durch die Primer im wesentlichen im Annealing an das Originaltarget oder/und in der folgenden Verlängerung der Primer; die weitere Amplifikation der vorhandenen PCR-Produkte ist durch die Zusatzveränderungen separiert.

In Fig. 2 ist die relative Lage der in den Beispielen eingesetzten Oligonukleotide angegeben (Position 1 am 3'-Ende). Es handelt sich hierbei um den Nachweis zweier Allele des Apolipoprotein-B-Gens (Proc. Natl. Acad. of Sciences USA 86, S. 587-591, 1989).

Zur zusätzlichen Separierung der Amplifikation der einzelnen Allele in einer Reaktion wurden die Oligonukleotide P1 und P2 durch weitere Y-Positionen an Pos. 28 (Y3:C, mismatch) und 29 (Y4:C, mismatch) von P2 unterschieden. Im folgenden ist die Angabe match bzw. mismatch auf die Sequenz des normalen Allels bezogen.
- Oligonukleotid P1 :: Asa kurz (29 Basen): SEQ ID No. 1
Nukleotid in Position X: T (selektiv mutant)
Nukleotid in Position 2 (Y1): G (mismatch)
Nukleotid in Position 4 (Y2): G (match)
- Oligonukleotid P2 :: Asa lang (49 Basen): SEQ ID No. 2
Nukleotid in Position X: C (selektiv normal)
Nukleotid in Position 2 (Y1): C (match)
Nukleotid in Position 4 (Y2): T (mismatch)
- Oligonukleotid P3 :: Gegenstrangprimer : SEQ ID No. 3
komplett komplementär zum Strang
- Oligonukleotid P2':: Asa lang' (49 Basen): SEQ ID No. 4
Nukleotid in Position X: C (selektiv normal)
Nukleotid in Position 2 (Y1): G (mismatch)
Nukleotid in Position 4 (Y2): G (match)

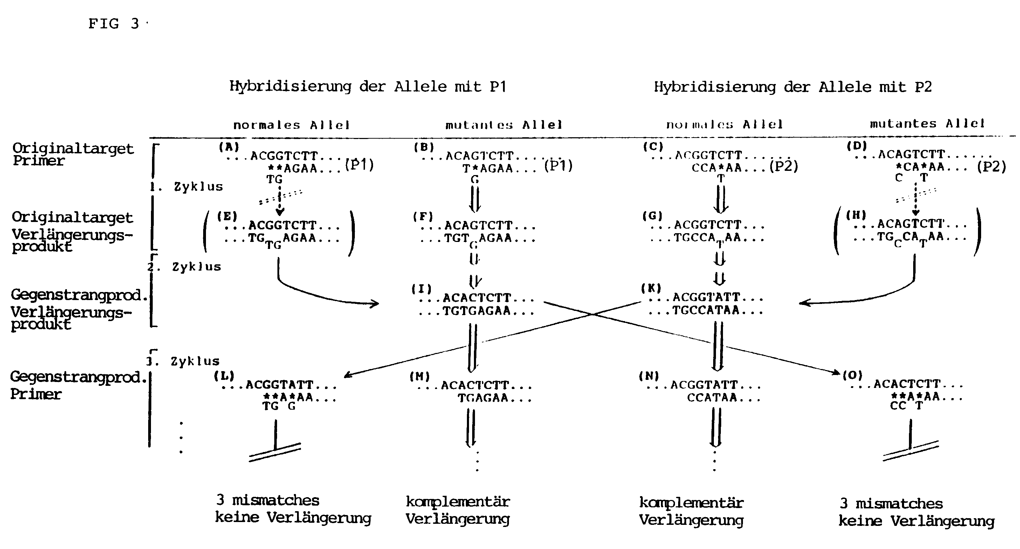

In Fig. 3 sind die Ergebnisse der ersten drei Zyklen einer PCR wiedergegeben, wenn die erfindungsgemäßen Oligonukleotide P1 und P2 in einem Ansatz zur Amplifikation benutzt werden. Die letzte Base am 3'-Ende des Oligonukleotids P1 paßt zum mutanten Allel, die letzte Base am 3'-Ende des Oligonukleotids P2 paßt zum normalen Allel, sodaß eine Verlängerung des Primers bei den in Abbildung 3 als (B) und (C) gekennzeichneten Primer/Template-Kombinationen erfolgt. Produkte der Reaktion sind die Nukleinsäurehybride (F) und (G). Die in (B) und (C) mit * gekennzeichneten Unterschiede (Mismatchs) verhindern die Verlängerung der Primer nicht vollständig. Im zweiten PCR-Zyklus bilden sich zu den Verlängerungsprodukten exakt komplementäre Gegenstränge (I) bzw. (K), obere Sequenz). Diese neu entstandenen Produkte können zwar mit dem jeweils anderen Oligonukleotid zu den Produkten (O) bzw. (L) hybridisieren, eine Verlängerungsreaktion jedoch findet nicht statt, da in diesen Hybriden 3 Mismatchs vorhanden sind. Dagegen findet eine Verlängerung der entsprechenden zugehörigen Oligonukleotide aus den Produkten (M) und (N) statt.

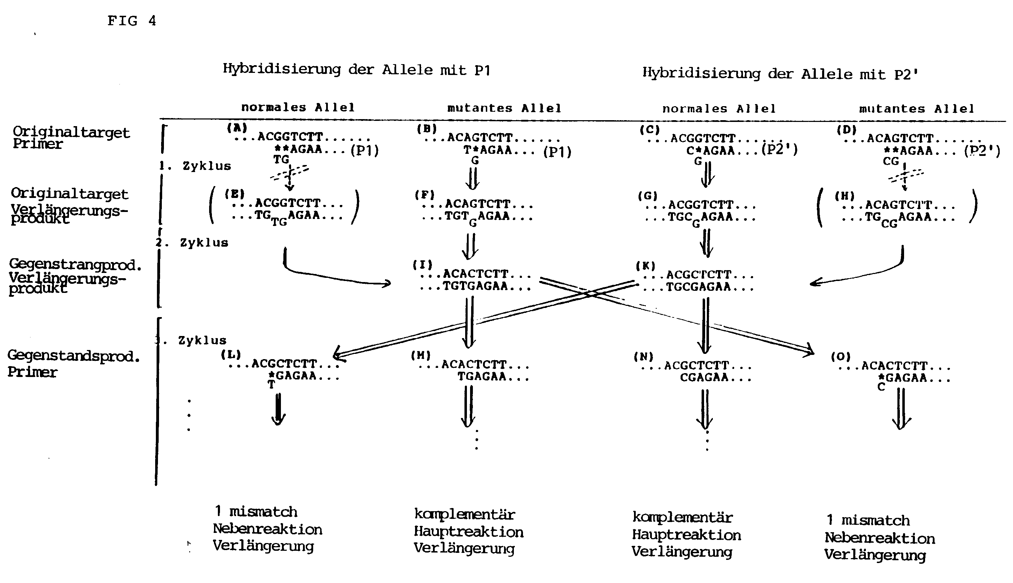

In Fig. 4 ist gezeigt, daß die Positionen Y der nicht komplementären Nukleotide bei verschiedenen Oligonukleotiden P1 und P2 nicht identisch sein dürfen, da sonst eine Nebenreaktion die Empfindlichkeit des Tests stark verringert. In Fig. 4 ist der Fall gezeigt, daß die beiden Oligonukleotide P1 und P2' jeweils ein Mismatch in den ersten beiden Positionen des in Verlängerungsrichtung liegenden Endes aufweisen. Es ist klar ersichtlich, daß im zweiten Zyklus der PCR Produkte gebildet werden, welche mit dem jeweils anderen Oligonukleotid nur ein einziges Mismatch aufweisen (Produkte O und L). An diesen Produkten kann eine die Spezifität herabsetzende Verlängerungsreaktion stattfinden, da die Produkte nur jeweils einen Mismatch aufweisen. Es wäre daher mit dem bekannten Verfahren nicht möglich, die beiden Allele mit ausreichender Spezifität in einem Reaktionsansatz zu bestimmen.

Die Spezifität des Nachweises bei Variante 2 kann noch durch zusätzliche differentielle Hybridisierung der Oligonukleotide an die nachzuweisenden Nukleinsäuren nach Art der COP gesteigert werden. In den bisher geschilderten Ausführungsformen von Variante 2 hybridisierten die Oligonukleotide mit allen nachzuweisenden Nukleinsäuren ungefähr mit gleicher Effizienz. Weisen jedoch die Oligonukleotide weitere Unterschiede in Position Y (z.B. mismatchs) auf, so kann durch geschickte Wahl der Temperatur der Hybridisierung der Oligonukleotide an die nachzuweisenden Nukleinsäuren z.B. bei der PCR (knapp unterhalb des T_{M}-Werts des Hybrids aus nachzuweisender Nukleinsäure und zugehörigem Oligonukleotid (match-Oligonukleotid) eine weitere Spezifitäts- und Ausbeuteerhöhung der Amplifikation erreicht werden. Diese zusätzlichen Y-Positionen sind nicht auf einen bestimmten, etwa den 3'-endnahen Bereich beschränkt, sondern können irgendwo im Oligo gewählt werden, wobei nach Definition der Y-Positionen sich die Oligonukleotide in diesen Positionen unterscheiden. Die Veränderungen jedes Oligonukleotids werden ins jeweilige Match-Produkt und das entsprechende Gegenstrangprodukt eingebaut und erschweren somit bei geeigneter Temperatur dem jeweils anderen Oligonukleotid das Annealing am falschen Produkttarget. Das Annealing des match-Oligonukleotids kann deshalb kaum noch durch ein Annealing des mismatch-Oligonukleotids am Produkttarget behindert werden, sodaß also eine Separierung der Amplifikation eintritt und eine Reduzierung der Amplifikationsrate durch blockierendes falsches Annealing ausgeschlossen ist.

In weiteren Ausführungsformen, insbesondere von Variante 2, können 2 oder mehr Nukleinsäuren mit 2 oder mehr Unterschieden (polymorphe Orte, X1, X2...XN) in der Nukleotidsequenz nachgewiesen werden, wobei die in (Nucl.Acids Res. 1988, Vol. 16, 23, 11141-11155) beschriebene Methode übertragen eingesetzt werden kann (Multiplex-Verfahren).

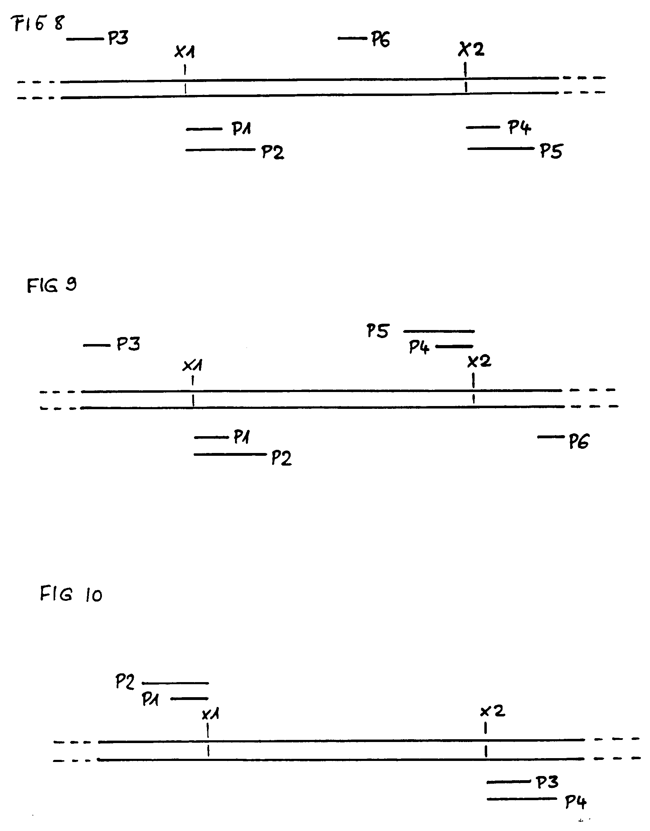

Es gibt dabei erfindungsgemäß mehrere Möglichkeiten. In einer ersten Ausführungsform werden die polymorphen Orte getrennt zum Gegenstand des erfindungsgemäßen Verfahrens gemacht. Es werden dann soviele erfindungsgemäße Oligonukleotide eingesetzt, wie polymorphe Orte auf verschiedenen Nukleinsäuren nachgewiesen werden sollen. Für 2 polymorphe Orte auf 2 Nukleinsäuren wird daher mindestens ein Satz von 4 erfindungsgemäßen Oligonukleotiden P1, P2, P4, P5 eingesetzt. Ein solcher Fall ist in Fig. 8 gezeigt. Die amplifizierten Bereiche die auch durch die beiden Gegenstrangprimer begrenzt werden, überlappen hier nicht, sind jedoch bevorzugt verschieden lang. Dadurch ist ein getrennter Nachweis einfach möglich. Alternativ kann auf Primer P6 verzichtet werden. Dann wird in der Amplifikation für beide polymorphe Orte der Gegenstrangprimer P3 genutzt.

Eine zweite Ausführungsform beruht darauf, daß die zu verschiedenen Orten gehörigen Sätze von erfindungsgemäßen Oligonukleotiden mit verschiedenen Strängen der nachzuweisenden Nukleinsäuren hybridisieren (Fig. 9). Dabei können die amplifizierten Bereiche auch überlappen, wobei durch analoge Nutzung von Y-Positionen (im überlappenden Bereich der Oligonukleotide P1, P2, P4 und P5) sichergestellt werden kann, daß die Amplifikation der beiden polymorphen Orte separiert bleibt. Auch hier sind beispielhaft 4 Oligonukleotide (P1, P2, P4 und P5) und zwei Gegenstrangprimer erforderlich.

Eine dritte, bevorzugte Ausführungsform (Fig. 10), bei der der Nachweis von Polymophismen miteinander gekoppelt ist, benötigt keine Gegenstrangprimer, da die erfindungsgemäßen Oligonukleotide als solche wirken. Auf einfache Weise können hier vier verschiedene Amplifikationsprodukte gebildet und nachgewiesen werden, je nachdem, ob entsprechende Allele vorhanden sind. Somit ist auch eine direkte Bestimmung der cis/trans-Lokation von Mutationen mögich.

Die obigen Ausführungsformen sind analog auch für mehr als zwei polymorphe Orte verwendbar.

Eine besonders bevorzugte Ausführungsform beruht auf der Verwendung der PCR (EP-A-0 201 184), worauf im folgenden inhaltlich Bezug genommen wird. Zum Nachweis von ähnlichen Nukleinsäuren ist es nur erforderlich, einen Primer durch einen Satz der erfindungsgemäßen Oligonukleotide zu ersetzen. Die übrigen Reaktionsbedingungen für die Amplifikation, insbesondere die Verwendung des Gegenstrangprimers, können analog angewendet werden.

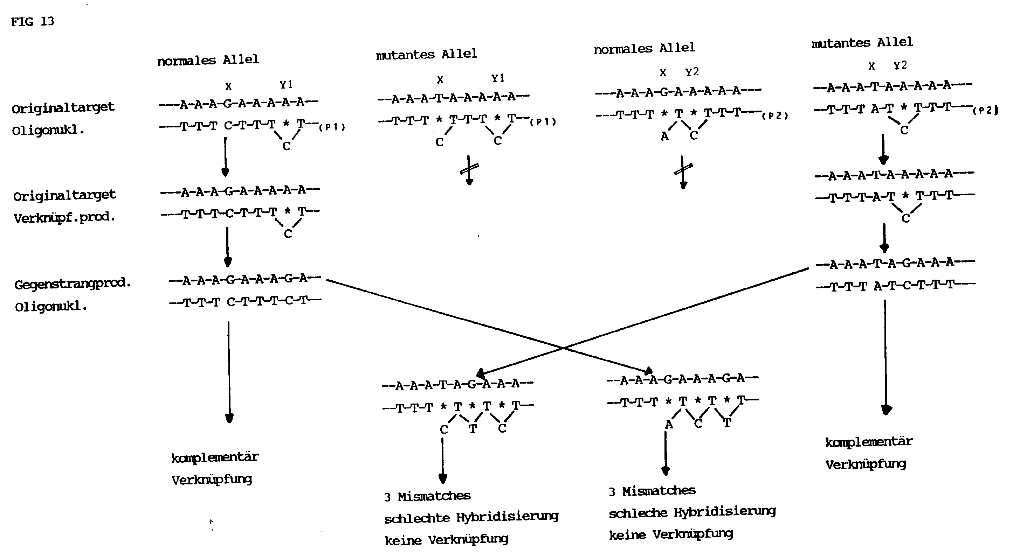

Auch die sogenannte Ligase-Chain-Reaction (LCR, EP-A-0320308) kann gemäß der Erfindung verbessert werden. Auch hier wird anstelle mindestens eines Oligonukleotids ein Satz von erfindungsgemäßen Oligonukleotiden eingesetzt. In Fig. 13 ist gezeigt, daß sich dadurch die Anzahl der Mismatches bei Kreuzprodukten erhöht. Je nach Lage von Y wird dadurch die Verlängerung oder/und das Annealing der falschen Oligonukleotide weitgehend unterdrückt. Position X kann sich bei dieser Ausführungsform am 3'-Ende des einen oder am 5'-Ende des anderen Oligonukleotids befinden. Im übrigen ist prinzipiell auch analog zur COP eine LCR durchführbar, bei der X nahe der Mitte des Oligonukleotids liegt. Bevorzugt werden als Gegenstrangoligonukleotide solche eingesetzt, die in den Y entsprechenden Positionen Nukleotide aufweisen, die zu keinem der Nukleotide der Positionen Y der erfindungsgemäßen Oligonukleotide komplementär sind.

Die erfindungsgemäße Verbesserung kann jedoch genauso in promotorgeprimten, zyklisch geführten Amplifikationsreaktionen eingesetzt werden, wie beim Verfahren der EP-A-0310229, der EP-A-0329822 oder der EP-A-0373960. Auf den Inhalt dieser Dokumente wird hiermit Bezug genommen. Bei diesen Amplifikationen wird mit Hilfe eines Promotorprimers eine Vielzahl von Produkttargets (RNA) gebildet, die erneut mit Hilfe der Promotorprimer amplifiziert werden.

Der spezifische Nachweis der Verlängerungsprodukte, welcher ein Maß für die Anwesenheit oder die Menge der nachzuweisenden Nukleinsäure in der Probe darstellt, ist beispielsweise dadurch möglich, daß sich die eingesetzten Oligonukleotide in einem weiteren Merkmal unterscheiden.

Der Unterschied kann beispielsweise darin liegen, daß die Oligonukleotide eines Satzes eine unterschiedliche Länge aufweisen. Dadurch entstehen durch die Verlängerung unterschiedlicher Oligonukleotide unterschiedlich lange Verlängerungsprodukte. Die Oligonukleotide können sich jedoch auch darin unterscheiden, daß sie unterschiedliche detektierbare Gruppen D enthalten. Solche detektierbaren Gruppen D sind beispielsweise Farb- oder Fluoreszenzmoleküle oder chemische Reste, die in einer nachfolgenden Reaktion mit einem nachweisbaren Rest bestimmt werden können. Solche chemischen Gruppen sind beispielsweise Haptene, wie Digoxin oder Digoxigenin. Haptene können detektiert werden, indem sie mit einem markierten Antikörper gegen das Hapten umgesetzt werden und die Markierung gemessen wird. Ein anderes Hapten könnte beispielsweise Biotin sein, welches mit anders markiertem Antikörper oder mit Streptavidin detektierbar gemacht wird.

Die verschiedenen Verlängerungsprodukte, welche ein Maß für die Menge und Anwesenheit der nachzuweisenden Nukleinsäure sind, können entweder nach Teilung des Ansatzes getrennt, bei Erhalt des Ansatzes sequentiell und bei Einsatz geeigneter Markierungen auch simultan nach bekannten Methoden nachgewiesen werden. Für die Verwendung von Steroidhormonen als Marker hat sich die in der EP-A-0 324 474 beschriebene Methode als bevorzugt erwiesen. Eine andere Methode besteht in der Verwendung unterschiedlicher Fluoreszenzfarbstoffe. Die Oligonukleotide können entweder direkt markiert werden, oder z.B. Antikörper gegen chemische Gruppen könnten entsprechend fluoreszenzmarkiert sein. Der Nachweis der verschiedenen allelischen Produkte ist dann durch simultane Fluoreszenzmessung in mehreren Kanälen möglich. Eine Markierung eines Teil der Produkte mit Fluoreszenzmarkern und anderer Produkte aus demselben Ansatz mit Enzymen ist ebenfalls möglich. Alle anderen denkbaren Markierungsmethoden und Detektionsverfahren sind prinzipiell ebenfalls geeignet.

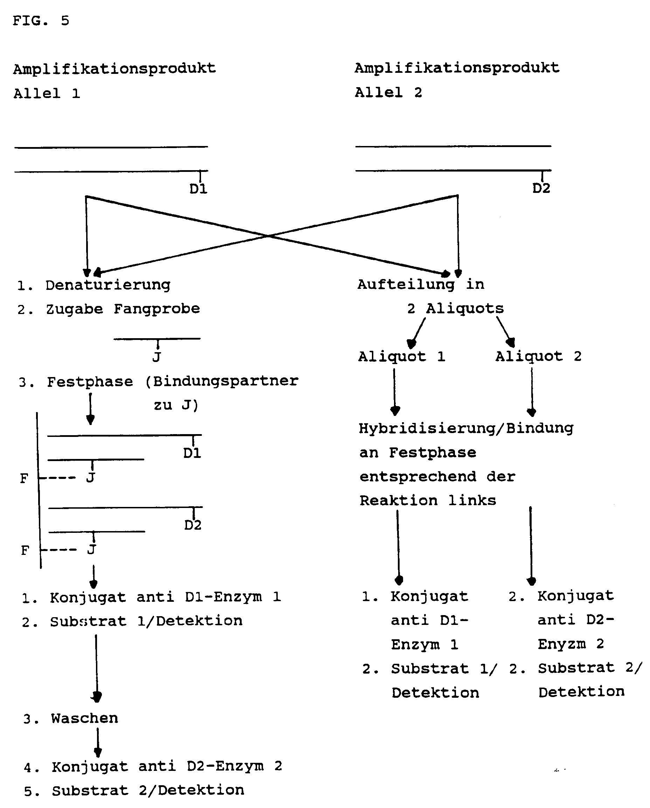

Bei sequentiellem oder simultanen Nachweis können beispielsweise 2 unterschiedliche chemische Gruppen, bevorzugt Haptene, die durch verschiedene Antikörper erkannt werden, zur Markierung der beiden Oligonukleotide verwendet werden. Beispiele für solche Haptene sind Digoxigenin, Biotin und Fluroeszein. Der Antikörper gegen Fluoreszein und der Antikörper gegen Digoxigenin haben dann bevorzugt unterschiedliche Enzyme als Markierung. Durch sequentielles oder simultanes Inkontaktbringen mit für die Enzyme spezifischen detektierbaren Substraten, können beide Nukleinsäuren nachgewiesen werden. Alternativen, wie die Verlängerungsprodukte nachgewiesen werden können, sind in Fig. 5 bis 7 angegeben.

Das Gemisch der gemeinsamen Verlängerungsreaktion wird in der in Fig. 5 (linke Seite) gezeigten Ausführungsform einer gemeinsamen Denaturierungsreaktion unterzogen. Die einzelsträngigen Verlängerungsprodukte werden mit einer immobilisierten oder über eine Gruppe I (z.B. Biotin) immobilisierbaren, sogenannten Fangsonde an eine Festphase F gebunden. Dabei werden die Verlängerungsprodukte der Reaktion mit allen nachzuweisenden Nukleinsäuren immobilisiert. Durch die unterschiedlichen detektierbaren Gruppen ist eine sequentielle Detektion nachzuweisender Nukleinsäuren (getrennt durch einen Waschschritt) möglich. Wenn die schließlich zu detektierenden Signale auch in einem Gefäß nebeneinander erzeugt und nachgewiesen werden können, kann auf den Waschschritt verzichtet werden.

Auf der rechten Seite von Fig. 5 ist eine Variante gezeigt, bei der nach der gemeinsamen Verlängerungsreaktion dem Reaktionsgemisch mindestens zweimal (oder entsprechend häufiger bei Nachweis von mehr als 2 Allelen und Verwendung von mehr als 2 Markierungsenzymen) eine ausreichende Menge an Flüssigkeit entnommen und diese einzeln auf die Verlängerungsprodukte geprüft werden. Aus einer Multiplexreaktion können Aliquots entnommen werden und je nach Zahl der verwendeten unterschiedlichen Label an den Oligonukleotiden können entsprechend viele Amplifikationsprodukte je Aliquot nachgewiesen werden.

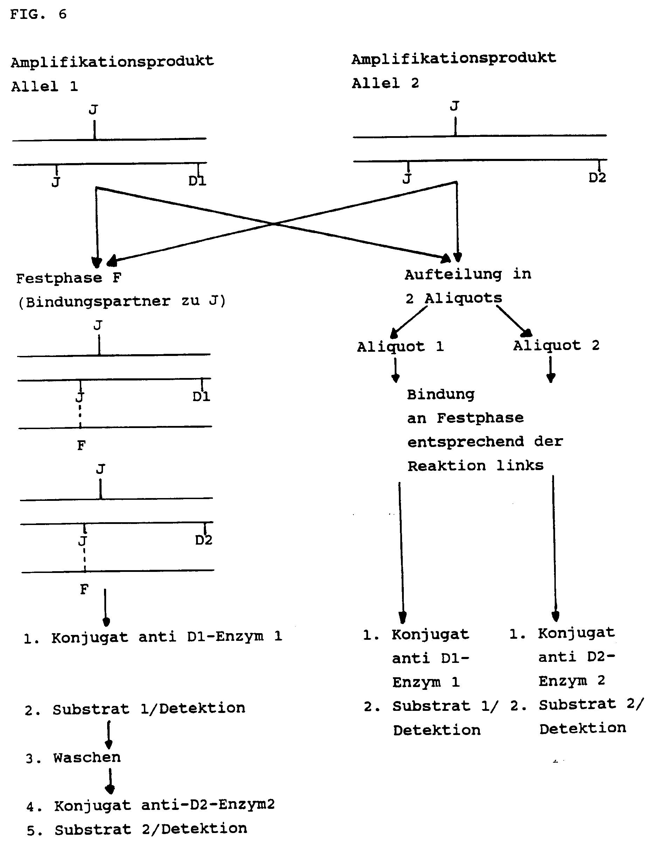

In Fig. 6 ist ein Verfahren abgebildet, bei dem bei der Verlängerungsreaktion auch ein mit einer immobilisierbaren Gruppe modifiziertes Mononukleotid oder Oligonukleotid eingebaut wurde. Dadurch können die Verlängerungsprodukte direkt (d.h. ohne separate Fangsonde und ohne Denaturierung) an eine Festphase gebunden werden (analog zu DE-A-4001145). Die Detektion ist ebenfalls wie in Fig. 5 gezeigt, simultan, sequentiell oder nebeneinander möglich.

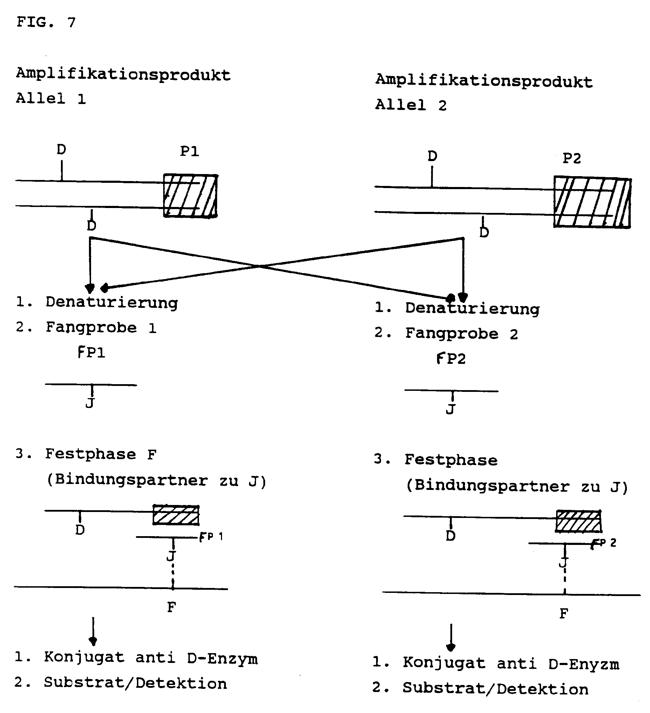

In Fig. 7 ist eine Ausführungsform gezeigt, die auf der Tatsache beruht, daß sich die Verlängerungsprodukte für verschiedene nachzuweisende Nukleinsäuren bevorzugt um mindestens drei Nukleotide voneinander unterscheiden. Dies kann für eine selektive Immobilisierung über eine Fangsonde ausgenutzt werden. Detektierbare Gruppen D werden durch Einbau eines modifizierten Mono- oder Oligonukleotids eingeführt. Diese Gruppe kann für die Verlängerungsprodukte beider Allele identisch sein. Wurde die Amplifikation der Nukleinsäuren nach der Variante 2 durchgeführt, so liegen die Variationen Y in der Nähe von X und die Fangsonde kann identisch zu einem Teil der Oligonukleotidsequenz und zu einem Teil der Verlängerungssequenz gewählt werden; damit erfolgt zugleich eine Selektion auf das richtige Verlängerungsprodukt (Artefakten fehlt die Verlängerungssequenz) und auf die verschiedenen Allele.

Weitere Ausführungsformen sind denkbar, beispielsweise über selektive Immobilisierung über eingebaute Oligonukleotide, die für jedes Allel eine andere immobilisierbare Gruppe aufweisen. Der Nachweis kann dann über eingebaute detektierbare Mononukleotide, einen eingebauten detektierbaren Gegenstrangprimer oder mittels einer detektierbaren Nachweissonde geschehen.

Der Nachweis bei Einsatz verschieden langer Oligonukleotide beruht bevorzugt auf gelchromatographischer Trennung der Verlängerungsprodukte, die so lang sind wie der amplifizierte Bereich (inklusive der Oligonukleotide). Die Detektion kann direkt nach Auftrennung im Gel, (z.B. Ethidiumbromidfärbung), nach Einbau markierter Oligonukleotide, Mononukleotide oder Hybridisierung mit markierten Sonden geschehen. Ein Unterschied in der elektrophoretischen Mobilität kann auch auf andere Weise erreicht werden, z.B. durch 5'-Endmarkierung eines Oligonukleotids mit Biotin; nach der Amplifikation wird ein Aliquot des Produktes mit Streptavidin inkubiert und einer Elektrophorese unterworfen: durch Bindung des Streptavidins an das biotinmarkierte Produkt wird die elektrophoretische Mobilität dieses Produkts deutlich reduziert, während das andere Produkt sich normal verhält.

Sofern eine der ähnlichen Nukleinsäuren in der Probe nicht enthalten ist, wird für diese Nukleinsäure bevorzugt kein Signal gemessen, welches durch Verlängerung des ihr entsprechenden Oligonukleotids hervorgerufen würde. Mit dem erfindungsgemäßen Verfahren kann daher auch nur eine der ähnlichen Nukleinsäuren nachgewiesen werden, oder auch die Abwesenheit aller ähnlichen Nukleinsäuren festgestellt werden.

Dadurch, daß keine Kreuzreaktionen des Verlängerungsprodukts aus einem Allel mit Oligonukleotiden, die zum Nachweis des anderen Allels eingesetzt werden, stattfinden, ist es möglich, die Amplifikation und gewünschtenfalls auch den Nachweis von ähnlichen Nukleinsäuren in einem Ansatz simultan und in einem einzigen Reaktionsgefäß vorzunehmen. Dies ist nicht in optimalem Umfang möglich, wenn die Positionen der Inkomplementarität Y (Mismatches) mit den nachzuweisenden Nukleinsäuren für verschiedene Oligonukleotide identisch sind.

Das erfindungsgemäße Verfahren kann vielseitig angewendet werden. Beispielsweise kann das Auftreten von Allelen in einer Probe, die von einem einzelnen Probanden gezogen wurde (Individualdiagnostik), in Korrelation zu bestimmten Erkrankungen (z.B. Stoffwechselstörungen) stehen.

Darüber hinaus ist das Verfahren wegen der guten Spezifität auch für Verfahren geeignet, die auf dem Prinzip des Pool-Screenings von Proben beruhen. Dazu werden eine große Anzahl von Individualproben verschiedener Versuchspersonen gemischt. Für die Diagnose des Defekts im apo B 3500-Gen hat es sich beispielsweise als zweckmäßig erwiesen, 64 Proben in einem Pool zu vereinen. Mit dem erfindungsgemäßen Verfahren ist es möglich, festzustellen, ob eine Probe des Pools den Gen-Defekt aufwies. Durch mehrmals hintereinander geschaltete Durchführung des erfindungsgemäßen Verfahrens mit Teilpools eines Pools mit positivem Resultat kann in, verglichen mit dem Stand der Technik, wenigen Bestimmungen die Probe ermittelt werden, welche das Gen mit dem Defekt aufweist.

Bei dem erfindungsgemäßen Verfahren erweist es sich als besonders vorteilhaft, daß gleichzeitig immer das Gen ohne den Defekt nachgewiesen werden kann, wenn es vorhanden ist. Beim Poolscreening kann alternativ das mutante Produkt nachgewiesen werden oder, wenn kein mutantes Allel vorhanden ist, das normale Produkt nachgewiesen werden. Die Möglichkeit, beide Allele nachweisen zu können, stellt neben einer Kontrollmöglichkeit der Reaktion als solche auch ein Mittel zur Quantifizierung der nachzuweisenden Nukleinsäuren dar.

Pool-Screening-Verfahren können wegen der gewährleisteten Probenanonymität besonders bei epidemiologisch interessanten Krankheiten, wie der Durchseuchungsrate mit dem AIDS-Virus oder der Durchsetzung von Bevölkerungsgruppen mit z.B. Hypercholesterinämie, Hypertonie oder Diabetes (Massenscreening) eingesetzt werden. Die Häufigkeit des Auftretens von Allelen kann damit bestimmt werden.

Dasselbe gilt für die Spezies-Diagnostik von Bakterien bzw. gegebenenfalls für die Virus-Diagnostik. Hier ist besonders der gleichzeitige Nachweis nahe verwandter pathogener/nicht pathogener Spezies bzw. Stämme interessant. Auch zum sicheren Nachweis von E.coli K12 (Beurteilung über das Gentechnologiegesetz) über die bekannten AT-Sequenzvarianten mit Unterscheidung von E.coli B-Stämmen ist das erfindungsgemäße Verfahren geeignet. Es kann somit in der Umweltanalytik angewendet werden.

Hauptvorteil der Erfindung gegenüber dem Stand der Technik ist die Möglichkeit, allele Verbindungen in Gemischen bei praktisch vernachlässigbarer Hintergrundreaktion gleichzeitig in einem Gefäß detektieren zu können. Ein Restriktionsverdau ist nicht erforderlich. Das Ziel wird erreicht durch eine quasi-Separierung der Amplifikationen (Verminderung von Kreuzreaktionen) der einzelnen Allele, obwohl eine Amplifikation in einem gemeinsamen Gefäß durchgeführt wird.

Ebenfalls Gegenstand der Erfindung ist ein Satz von Oligonukleotiden, welcher mindestens zwei Oligonukleotide enthält, die mit ähnlichen Nukleinsäuren hybridisieren können, wobei sich die Oligonukleotide in mindestens 2, bevorzugt mindestens in 3 definierten Positionen sowie bevorzugt einem weiteren Strukturmerkmal unterscheiden. Bevorzugt sind solche Oligonukleotide, die sich in ihren 3'-endständigen Nukleotiden (die Position X entsprechen) und in jeweils zwei weiteren Nukleotiden sowie bevorzugt einem weiteren Strukturmerkmal voneinander unterscheiden. Das detektierbare Strukturmerkmal kann die unterschiedliche Länge der Oligonukleotide oder auch eine nachweisbare oder immobilisierbare Gruppe sein. Die Oligonukleotide können als Gemisch vorliegen. Außerdem kann dieses Gemisch noch weitere Oligonukleotide enthalten, die bei der Amplifizierung von Nukleinsäuren mit Hilfe des erfindungsgemäßen Satzes von Oligonukleotiden erforderlich sind, insbesondere Gegenstrangoligonukleotide bzw. -primer.

Ebenfalls einen erfindungsgemäßen Effekt zeigen Oligonukleotide, die in Position Y unterschiedliche Nukleotide aufweisen, wobei diese aber jeweils nicht zu den Nukleotiden der Templatnukleinsäuren komplementär sind, wenn diese Position bei den Oligonukleotiden dieses Satzes den gleichen Abstand von X hat. Einen abgeschwächten Effekt zeigt ein solches Set von Oligonukleotiden, von denen nur eines in der Position Y ein Nukleotid aufweist, das nicht zu der entsprechenden Position der zugehörigen nachzuweisenden Nukleinsäure komplementär ist. Der erfindungsgemäße Satz von Oligonukleotiden wird zur Lösung der Aufgabe in dem erfindungsgemäßen Verfahren verwendet.

Ein weiterer Gegenstand der Erfindung ist ein Reagenzkit zum Nachweis von ähnlichen Nukleinsäuren, welches einen erfindungsgemäßen Satz von Oligonukleotiden sowie ein Enzym zur Verlängerung von Nukleinsäuren und ggf. weitere zur Verlängerung der Oligonukleotide erforderliche Reagenzien und Hilfsstoffe enthält. Außerdem kann das Reagenzkit weitere Oligonukleotide, beispielsweise Gegenstrangprimer, enthalten. In dem Reagenzkit liegen bevorzugt das Enzym und die Oligonukleotide in von den übrigen Reagenzien getrennten Behältern vor.

Weiterer Gegenstand der Erfindung sind die Verwendung des erfindungsgemäßen Verfahrens zur gleichzeitigen Bestimmung von Allelen in einer Probe (z.B. auch zur Typisierung von Transplantationsantigenen auf DNA-Basis), zur gleichzeitigen Bestimmung von topologisch nahe verwandten Infektionserregern (z.B. auch in der Verlaufs- und Therapiekontrolle) und zum Screening von Proben-Pools auf die Anwesenheit von nahe verwandten Nukleinsäuren oder deren Häufigkeit. Außerdem wird die Verwendung des Verfahrens zur Ermittlung von Mutationen beansprucht.
Fig. 1 zeigt in theoretischer Form ein erfindungsgemäßes Verfahren, welches auf kompetitivem Priming beruht.
Fig. 2 zeigt die Anordnung von Nukleotidsequenzen, wie sie Fig. 3 sowie den Beispielen zugrundeliegt.
Fig. 3 zeigt das erfindungsgemäße Verfahren zur Verwendung von 3'-Mismatch-Oligonukleotiden.
Fig. 4 zeigt ein Verfahren bei dem die Mismatch-Nukleotide beide in der gleichen Position nahe dem Ende der Oligonukleotide lokalisiert sind.
Fig. 5 bis 7 zeigen Ausführungsformen zur Detektion der Verlängerungsprodukte des erfindungsgemäßen Verfahrens.
Fig. 8 bis 10 zeigen die Anwendung des erfindungsgemäßen Verfahrens zum gleichzeitigen Nachweis mehrerer polymorpher Orte.
Fig. 11 zeigt eine Analyse der in Beispiel 1 amplifizierten Proben.
Fig. 12 zeigt die Analyse der im Beispiel 2 amplifizierten Proben und einer negativen Kontrolle.
Fig. 13 zeigt das erfindungsgemäße Verfahren unter Verwendung einer LCR.

### Bezugszeichenliste

- I: Gruppe für Immobilisierung
- D: Gruppen für Detektion
- D1, D2: unterschiedliche Gruppen für Detektion
- F: Festphase
- P1, P4: Primer 1, 4 gemäß der Erfindung
- P2, P5: Primer 2, 5 gemäß der Erfindung
- P2': Vergleichsprimer
- P3, P6, P7: Gegenstrangprimer
- X, X1, X2: allele Positionen
- Y1, Y2: Positionen der Mismatches

Die folgenden Beispiele dienen der Verdeutlichung der Erfindung.

### Beispiel 1

### Nachweis der beiden Allele bezüglich der CGG--->CAG-Mutation im Codon 3500 des Apolipoprotein B-Gens in einer Reaktion

### Probenvorbereitung

Humane genomische DNA wurde aus EDTA-Blut nach der Methode von Higuchi (in PCR Technology, Principles and Application for DNA Amplification, ed. Ehrlich, H.A. (Stockton press, New York), 31-38 (1989)) isoliert, wobei das am Ende erhaltene Volumen an DNA-Lösung gleich dem Volumen des eingesetzten EDTA-Blutes ist. Bei im Mittel 7500 Leukocyten/µl wird beispielsweise eine 2 µl Probe bis zu 15.000 Leukocyten enthalten, sodaß mit bis ungefähr 100 ng DNA in je 2 µl Lösung gerechnet werden kann. Die DNA wurde am Ende der Methode von Higuchi auf 100°C erhitzt, sodaß eine weitere Denaturierung vor Durchführung der PCR nicht mehr erforderlich ist.

### Erfindungsgemäße Amplifikation mit Hilfe der PCR

Die PCR gemäß EP-A-0 200 362 wurde in 0,5 ml Tubes (Sarstedt Nr. 72699) und einem GENE-ATAQ-Controller (Pharmacia) unter Verwendung des folgenden Reagenzgemischs durchgeführt:
10 µM jeweils dATP, dGTP, dTTP, dCTP
1 mM MgCl₂
6 µl 10 x PCR-Puffer ohne Mg²⁺
0,6 µl Asa kurz (Primer P1; 95.7 ng/µl), SEQ ID NO 1
1 µl Asa lang (Primer P2; 81 ng/µl), SEQ ID NO 2
0,6 µl Gegenstrang Primer P3 (92.4 ng/µl), SEQ ID NO 4
0.3 µl Taq-Polymerase (5 Units/µl, Beckmann)
60 µl Gesamtvolumen

Der 10 x PCR-Puffer ist: 100 mM Tris-HCl pH 8.3, 500 mM KCl, 0,1% Gelatine

### Temperaturverlauf:

45mal (1 Min. 95°C, 1 Min. 65°C, 1 Min. 72°C)
4mal (2 Min. 65°C, 1 Min. 72°C)
1mal (5 Min. 72°C)
   Abkühlung auf Raumtemperatur

### Detektion der Verlängerungsprodukte

10 µl der PCR-Produkte wurden anschließend mit 2 µl Auftragpuffer (40% Sucrose, 0,25% Bromphenolblau, 0,25% Xylenecyanol, 0.1 M EDTA pH 8.0) gemixt und in einem Agarosegel elektrophoretisch untersucht. Die Zusammensetzung des Gels war 3% NuSieve GTG (FMC/Biozym), 1% Agarose NA (Pharmacia), 1 x TAE, 0,5 µg/ml Ethidium-Bromid; (50 x TAE = 242g Tris Base, 57,1 ml Eisessig, 100 ml 0,5 M EDTA pH 8.0). Die Laufzeit der Gele beträgt 0,5 -1 h bei ca. 5 V/cm. Nach der Photographie der Fluoreszenz bei 300 nm mit Polaroid 667 black & white instant film unter Verwendung einer Camag Reprostar Einrichtung, erfolgt die Auswertung anhand des Photos.

Um zu demonstrieren, daß hier die beiden allelselektiven Primer nötig sind zur sicheren Identifizierung der Allele, wurden folgende Proben analysiert (s. Fig. 11):
1: Längenstandard
2: heterozygote Probandin
3: homozygot normaler Proband und
4: kloniertes mutantes Allel, Menge (molar) entsprechend Probe 3

Die Proben wurden unter Verwendung der beiden allelselektiven Primer P1, P2 und des Gegenstrangprimers P3 amplifiziert. Die normale Probe liefert, wie erwartet, nur ein 134 bp Produkt, die mutante Probe nur ein 114 bp Produkt. Die heterozygote Probandin (Spur 4 in Fig. 11) liefert dagegen, wie erwartet, beide Produkte. Wird dagegen nur einer der allelselektiven Primer eingesetzt, erhält man das dem Primer entsprechend lange PCR-Produkt unabhängig davon, welches der Templates amplifiziert wurde. So liefert insbesondere der normale Proband auch die dem mutanten Allel entsprechende Bande (Spur 6) und die klonierte mutante DNA das Produkt des normal selektiven Primers (Spur 10). In diesem Fall ist also unter den gegebenen Reaktionsbedingungen eine Amplifikation mit dem ursprünglichen ARMS-Konzept (C.R. Newton et al., Nucleic Acids Research 17, 2503-2516 (1989)) nicht zur Unterscheidung der Allele geeignet. Erst die Verwendung der hier beschriebenen Erfindung erlaubt im vorliegenden Fall die korrekte und eindeutige (Spur 2, 3, 4) Bestimmung des Genotyps und sie erlaubt darüberhinaus die Bestimmung beider Allele in einem Ansatz.

Bei der klonierten DNA handelt es sich um ein Fragment, das die Positionen 10573-1070 der Apo B cDNA Sequenz umfaßt im Vektor Gem3zf(-) (Atlanta/Promega). Vor dem Einsatz in die PCR-Reaktion wurde die DNA linearisiert mit BamH1 und verdünnt auf 0.06 pg/µl. Diese Verdünnung enthält ca. 15000 DNA-Moleküle/µl, also in 2 µl etwa ebensooft die Apo B Sequenz wie 2 µl DNA-Lösung aus einer DNA Isolation aus EDTA-Blut.

Diese Versuche zeigen, daß die Entstehung unspezifischer Produkte, die trotz Zusatzmaßnahmen zur Spezifitätserhöhung bei Reaktionsführung in getrennten Gefäßen auftreten, durch Verwendung der erfindungsgemäßen Oligonukleotide in einem Reaktionsgefäß unterdrückt werden kann.

### Beispiel 2

### Screening für die CGG--->CAG-Mutation im Codon 3500 des Apolipoproteins B-Gens (Poolscreening)

Dieses Beispiel zeigt, daß die beschriebene Methodik zum Nachweis eines Carrierallels in einem Pool mit DNA anderer Probanden geeignet ist. Zunächst wurde die Sensitivität überprüft. Dazu wurde durch DNA Isolation nach Higuchi (s. Beispiel 1) gewonnene DNA wie folgt gemischt:
heterozygote DNA: normal homozygote DNA
1:50, 1:100, 1:200, 1:400 und 1:800
2 µl der erhaltenen DNA-Lösungen wurden in drei Reaktionsschritten amplifiziert:

### 1. Reaktionsschritt

In dieser Vorreaktion wurde nur der normalselektive Primer eingesetzt um die DNA, die bei Präparation denaturiert wurde, also einzelsträngig vorlag, wieder in Doppelstrang zu überführen. Dadurch wird ein falsches Primen durch den mutant-selektiven Primer am großen Überschuß normaler Allele vermieden, was bei der niedrigen Temperatur, bei der die PCR angesetzt wird, sonst leicht aufträte. Durch diese Vorreaktion ist sichergestellt, daß der mutant-selektive Primer nur bei der gewünschten Annealingtemperatur in den nachfolgenden Reaktionsschritten auf einzelsträngiges normales Template trifft. In dieser Vorreaktion ist die dNTP-Konzentration durch das relativ kleine Gesamtvolumen von nur 12 µl relativ hoch, da in diese Reaktion bereits die gesamte dNTP Menge eingesetzt wurde, die für den

### 2. Reaktionsschritt gebraucht wird. Dies erleichtert die vollständige Überführung der einzelsträngigen normalen Allele in Doppelstrang.

| |
|---|
| 2 µl DNA-Lösung |
| 10 µl Aliquot des Reagenzienmixes: |
| 1.4 µl dNTP Mix (20 µM Stammlösung) |
| 0.9 µl Mg²⁺ (20 mM Stammlösung) |
| 1.2 µl 10 x PCR-Puffer ohne Mg²⁺ (s. Beispiel 1) |
| 0.28 µl Primer Asa lang (81 ng/µl Stammlösung) SEQ |
| ID NO 2 |
| 5.99 µl H₂O |
| 0.23 µl Taq-Polymerase (5 U/µl, Beckmann) |
| 12 µl Gesamtvolumen |

Temperaturverlauf: 4 x (2 min 60°C, 2 min 72°C), 5 min 72°C, ohne Ölüberschichtung.

### 2. Reaktionsschritt

### PCR mit den Primern P1, P2 und dem Gegenstrangprimer P7 (5' zu Gegenstrangprimer P3 aus Fig. 2, SEQ ID NO 5).

Der zusätzliche Gegenstrangprimer wurde verwendet, um Primerdimerprobleme auszuschalten. Sowohl Primer P7 als auch Primer P3 liefern bei sehr großer Anzahl von Zyklen Primerdimere, wobei unter Umständen das gewünschte PCR-Produkt nicht mehr erhalten werden konnte. Durch eine Amplifikation mit Primer P7 (dieser 2. Reaktionsschritt) und anschließende weitere Amplifikation mit Primer P3 (Reaktionsschritt 3.) wird die Bildung der Primerdimere unterdrückt.

Die Konzentration des normalselektiven Primers ist in dieser Reaktion deutlich niedriger als die des mutantselektiven Primers, da nur die in der Reaktion 1 vorhandene Menge an normalselektivem Primer übernommen wurde. Dadurch ist eine bevorzugte Amplifikation des mutanten Allels möglich. Die vorhandene Menge an normalselektivem Primer reicht jedoch aus, für den Fall, daß kein mutantes Allel im Ansatz vorhanden ist, um ein sichtbares Amplifikationsprodukt nach dem 3. Reaktionsschritt zu erhalten und so auch im Poolscreening eine interne Kontrolle der PCR-Reaktion zu ermöglichen.

### Ansatz:

| |
|---|
| 12 µl = Ansatz des 1. Reaktionsschrittes |
| 58 µl Aliquot des Reagenzienmixes: |
| 1.4 µl Primer P7 (51.1 ng/µl Stammlösung) |
| SEQ ID NO 5 |
| 0.7 µl Asa kurz (95.7 ng/µl Stammlösung) |
| SEQ ID NO 1 |
| 0.85 µl Mg²⁺ von 20 mM MgCl₂ Lösung |
| 5.8 µl 10 x PCR-Puffer ohne Mg²⁺ |
| 0.12 µl Taq-Polymerase |
| 49.13 µl H₂O |
| 70 µl Gesamtvolumen |

Die Proben wurden mit ca. 50 µl heavy white mineral oil (Sigma Cat. No. 400-5) überschichtet.
- Temperaturverlauf:: 20 x (1 min 95°C, 1 min 60°C,
1 min 72°C),
4 x (2 min 60°C, 2 min 72°C)
5 min 72°C

Die letzten vier Zyklen (nur Annealing und Verlängerung, ohne Denaturierung) sollen sicherstellen, daß vor Ansetzen des dritten Reaktionsschrittes keine einzelsträngige DNA mehr vorliegt.

### 3. Reaktionsschritt

### PCR mit Gegenstrangprimer P3

| |
|---|
| 7 µl des Produkts des 2. Reaktionsschrittes |
| 43 µl Aliquot des Reagenzienmixes: |
| 0.5 µl Primer 3 (92.4 ng/µl) SEQ ID NO 4 |
| 0.65 µl Asa kurz, SEQ ID NO 1 |
| 0.2 µl Asa lang, SEQ ID NO 2 |
| 5 µl 10 x Puffer ohne Mg²⁺ |
| 2.14 µl Mg²⁺ von 10 mM MgCl₂ Lösung |
| 2.5 µl dNTP-Mix, in dem die vier dNTPs je 100 µM |
| vorliegen |
| 31.76 µl H₂O |
| 0.25 µl Taq-Polymerase (5 U/µl, Beckmann) |
| 50 µl Gesamtvolumen |

- Temperaturverlauf:: 40 x (1 min 95°C, 1 min 65°C,
1 min 72°C),
4 x (2 min 65°C, 2 min 72°C),
5 min 72°C

In der 2. Reaktion ist die Selektivität im wesentlichen durch die niedrige dNTP Konzentration (2 µM) bestimmt, während die Annealingtemperatur mit 60°C relativ niedrig ist, um ein effizientes Annealing zu gewährleisten. In der 3. Reaktion wird dagegen eine höhere dNTP-Menge eingesetzt, so daß eine größere Menge an Produkt gebildet werden kann. Da die Zusatzmutationen in den Primern mit der zweiten Reaktion in die PCR-Produkte eingebaut wurden, konnte die Annealingtemperatur in der 3. Reaktion auf 65°C erhöht werden.

Für die Anwendung zum Screening für die B3500 Mutation wurde das Schema zum Poolen der Proben der Häufigkeit der Mutation in der Allgemeinbevölkerung angepaßt. Die B3500 Mutation tritt ca. bei einer von 500 bis 700 Personen auf. Die Analyse der Proben sollte in drei Stufen erfolgen:
1. Analyse von relativ großen Pools,
2. Analyse von kleineren Pools, aus denen die größeren Pools gebildet wurden,
3. Analyse der einzelnen Proben.

Dazu wurden je 8 EDTA-Blutproben zu primären Pools gemischt und von je 8 primären Pools wurde ein Aliquot zur Erzeugung eines 64-er Pools verwendet. Dieses Schema ist optimal für eine dreistufige Analyse bei einer Häufigkeit der Mutation von 1 in 8³ also 1 in 512, kann aber auch über einen weiten Bereich anderer Häufigkeiten eingesetzt werden.

Bei der Mischung der EDTA-Proben wurde die unterschiedliche Konzentration der Leukozyten der einzelnen Proben berücksichtigt durch unterschiedliche Volumina, so daß je Proband eine gleiche Anzahl Leukozyten in die Pools einging. Zu diesem Zweck wurden zunächst 100 µl EDTA-Blut je Proband auf Mikrotiterplatten pipettiert, so daß die anschließenden Pipettierungen von einem Pipettierroboter übernommen werden konnten (Tecan RSP 5052, Zinsser Analytic, Frankfurt). Durch die Maschine wurde jede Probe zunächst mit 100 µl 0.9% NaCl verdünnt und Aliquots mit ca. 80000 Leukozyten (Volumina per Computer berechnet aus Leukozytenkonzentrationen) wurden zu den primären 8-er Pools vereinigt. Von den 8-er Pools wurden (nach Mischen durch die Maschine) je ca. 160000 Leukozyten zu 64-er Pools pipettiert. Die Isolation der DNA wurde zunächst nur für die 64-er Pools durchgeführt. Ca. 4100 Proben wurden auf diese Weise analysiert. Dabei wurden 6 64-er Pools gefunden, die mutante Sequenz enthielten. Die DNA der entsprechenden 8-er Pools wurde isoliert und in gleicher Weise analysiert wie die 64-er Pools. Schließlich wurde die DNA der zu den positiv gefundenen 8-er Pools gehörigen Einzelproben isoliert und entsprechend Beispiel 1 untersucht. Auf diese Weise wurden 6 heterozygote Carrier identifiziert.

Für die negative Kontrolle wurde nur die normale Bande erhalten. Mit geringfügig anders angesetzter Mischung der Oligonukleotide (etwas weniger ASA lang) kann man erreichen, daß man für negative Kontrollen stets auch eine etwas schwächere Bande an der Position des mutanten Produkts erhält. Damit ist das höchste Maß der Sensitivität dieses Systems erreicht, da nun bereits durch Fehlpriming bzw. Fehler der Taq-Polymerase bei der Gegenstrangsynthese entstandender Hintergrund gerade sichtbar wird. Damit ist sichergestellt, daß auch geringe Mengen an tatsächlich vorhandenem mutanten Original detektiert werden (dann verschwindet die normale Bande), sofern sie oberhalb des Hintergrundes liegen.

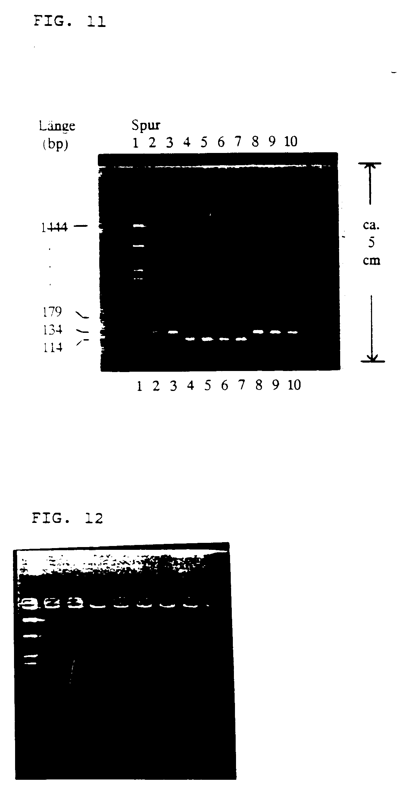

Fig. 12 zeigt die Analyse der amplifizierten Proben und einer negativen Kontrolle. Während bei der Negativkontrolle nur das lange PCR-Produkt auftritt (134 bp, Spur 2), findet man bei 3 und 4 nur das mutante PCR-Produkt (114 bp). Bei der Verdünnung 5 bis 7 findet man dominant das mutante PCR-Produkt, und auch einen sehr geringen Anteil des normalen Produkts. Die beschriebene Methode erlaubt es also, DNA eines heterozygoten Probanden auch noch in einer Mischung mit DNA 800 normaler Probanden nachzuweisen. Noch größere Verdünnungen wurden bisher nicht getestet.
1: Längenstandard
2: Negativkontrolle
3: 1:50
4: 1:100
5: 1:200
6: 1:400
7: 1:800

### Beispiel 3

### Verfahren unter Verwendung eines nicht verlängerbaren Oligonukleotids für eines der Allele

Besonders für Multiplexverfahren können gegebenenfalls auch Oligonukleotide gegen eine Verlängerung blockiert werden z.B. durch Einbau eines Dideoxynukleotids am 3'-Ende (Cozzarelli et al. Journal of Molecular Biology 45, 513 (1969) ). In einem Multiplexsystem, das dazu dient zu bestimmen, ob von mehreren bekannten (seltenen) Mutationen eine (oder mehrere) vorliegt, können so die normalen Allele von der Amplifikation ausgeschlossen werden, sodaß die Zahl der Amplifikationsprodukte für jeden Probanden in der Regel auf 1-3 Banden beschränkt bliebe (1 normale Bande wird mitamplifiziert zur Kontrolle der Amplifikation, 1-2 Banden mutante Allele). Ein falsches Annealing am Originaltemplate in Zyklen, die nach dem primären Annealingschritt liegen, kann zusätzlich durch anschließende Zugabe von blockierten Oligonukleotiden erfolgen, die perfekt komplementär zum Originaltarget sind.

Für den Fall, daß im groben die Konzentration eines Allels oder eines Isolats eines infektiösen Agens oder einer somatischen Mutante (Onkogene)) mit Variante 2 abgeschätzt werden soll, so kann ein drittes künstliches Allel als Standard eingesetzt werden und für dieses und das Allel, das abgeschätzt werden soll, wird ein erfindungsgemäßer Primer mit 3'-OH Ende eingesetzt, während die Amplifikation der übrigen Allele durch Dideoxyoligonukleotide verhindert wird. Das künstliche 3. Allel kann die Y-Abweichungen des entsprechenden Primer auch schon enthalten und insofern als der Primer dann direkt zum Template matched relativ viele Y-Positionen tragen um eine sichere Separierung der Amplifikationen zu erzielen.

Bei der Polymerisation von Nukleotiden zu Polynukleotiden erfolgt eine Verknüpfung zwischen der 5'-OH Gruppe und der 3'-OH Gruppe über eine Phosphodiesterbindung. Ein Dideoxynukleotid besitzt keine 3'-OH Gruppe. Eine Verlängerung einer Nukleinsäure mit Dideoxynukleotid am Ende ist also nicht möglich. Dieser Effekt wird ja z.B. beim Sequenzieren genutzt. Beim Multiplexing treten nun aber zahlreiche Banden auf, die zunächst eher verwirren. Z.B. wurde im Labor bei uns bereits ein Multiplexing für 5 Mutationen in einem Ansatz durchgeführt mit der Variante 2 (allelspezifische Primer). Dabei treten also mindestens 5 Banden auf (normale Person ohne Mutationen). Sofern eine Person für zwei Mutationen heterozygot ist sogar 7 Banden. Wenn aber für ein bestimmtes Gen (in diesem Falle LCAT, z.B. aber auch LDL-Rezeptor) und eine bestimmte Person nur überprüft werden soll, ob eine der bekannten Mutationen vorliegt, kann auf die Amplifikation der normalen Allele (außer einer Bande als PCR-Kontrolle) verzichtet werden. Dadurch reduziert sich die Zahl der Banden bei einer Normalperson auf eine Bande. Je vorhandene Mutation kommt nur eine Bande dazu. Die Gesamtausbeute der PCR verteilt sich also auf deutlich weniger Banden und das Bandenmuster wird vereinfacht. Hat man so z.B. von 40 verschiedenen möglichen Defekten einen nachgewiesen (in mehreren Multiplexreaktionen), so kann anschließend auf Heterozygotie oder Homozygotie getestet werden (diesmal statt mit dideoxy- mit normalem Primer). Um in einem solchen Multiplexing das normale Allel durch ein Oligo abzudecken, und so eine Hybridisation mit z.B. dem falschen COP-Primer auszuschließen, kann (je Mutationsstelle je) ein Dideoxyoligonukleotid eingesetzt werden. Durch im übrigen erfindungsgemäße Primer paßt anschließend der mutante Primer perfekt zu einem eventuell gebildeten PCR-Produkt vom mutanten Allel. Auch bei Variante 2 (allelspezifische Primer) könnte ein Dideoxyoligonukleotid eingesetzt werden, um ein fälschliches Annealing und Primern des anderen Primers zu reduzieren.

Bei LCR Reaktionen könnten analog 5'-OH Oligonukleotide eingesetzt werden.

Bei promotergesteuerten Varianten kann analog eines der obigen Oligonukleotide eingesetzt werden oder ein Primer, der eine nicht als Promoter wirksame Variante der Promoter-Sequenz enthält.

### Sequenzprotokolle

### SEQ ID NO 1

- Sequenzlänge:: 29 Basen
- Art der Sequenz:: Desoxyribonukleotid
- Strangform:: Einzelstrang
- Topologie:: Linear
- Position:: 10658-10686 (J. Biol. Chem. 261,12918 -12921 (1986))

3'-TGAGAAGTCA CTTCGACGTC CCGTGAAGG-5'

### SEQ ID NO 2

- Sequenzlänge:: 49 Basen
- Art der Sequenz:: Desoxyribonukleotid
- Strangform:: Einzelstrang
- Topologie:: Linear
- Position:: 10658-10706

3'-CCATAAGTCA CTTCGACGTC CCGTGAACCT TTTAACTACT ATAGACCTT-5'

### SEQ ID NO 3

- Sequenzlänge:: 49 Basen
- Art der Sequenz:: Desoxyribonukleotidsequenz
- Strangform:: Einzelstrang
- Topologie:: Linear
- Position:: 10658-10706

3'-CGAGAAGTCA CTTCGACGTC CCGTGAACCT TTTAACTACT ATAGACCTT-5'

### SEQ ID NO 4

- Sequenzlänge:: 28 Basen
- Art der Sequenz:: Desoxyribonukleotidsequenz
- Strangform:: Einzelstrang
- Topologie:: Linear
- Position:: 10573-10600

5'-GATGTCAAGG GTTCGGTTCT TTCTCGGG-3'

### SEQ ID NO 5

- Sequenzlänge:: 31 Basen
- Art der Sequenz:: Desoxyribonukleotidsequenz
- Strangform:: Einzelstrang
- Topologie:: Linear
- Position:: 10532-10562

5'-GCCTCACCTC TTACTTTTCC ATTGAGTCAT C-3'

## Patentansprüche

1. Verfahren zum Nachweis ähnlicher Nukleinsäuren, deren Nukleotidsequenzen sich in mindestens einer Position (X) voneinander unterscheiden, welches mindestens folgende Teilschritte enthält:
a) Umsetzung eines Satzes von Oligonukleotiden, deren Nukleotidsequenzen sich in der der Position X entsprechenden Position unterscheiden, mit den nachzuweisenden Nukleinsäuren unter Hybridisierungsbedingungen;
b) Verlängerung der anhybridisierten Oligonukleotide unter Verwendung der nachzuweisenden Nukleinsäuren als Template in einem einzigen Reaktionsgefäß; und
c) Nachweis von Verlängerungsprodukten;
dadurch gekennzeichnet, daß
- sich die Oligonukleotide des Satzes außerdem in mindestens einer weiteren Position (Y) voneinander unterscheiden, wobei die Positionen X und Y im hybridisierenden Bereich liegen, so daß sich die Verlängerungsprodukte aus b) in mehr als einer Position im hybridisierenden Bereich voneinander unterscheiden und
- in Schritt c) die in Schritt b) erzeugten Unterschiede der Verlängerungsprodukte zu den ähnlichen Nukleinsäuren sowie die Position (X) zum Nachweis der Verlängerungsprodukte verwendet werden.

2. Verfahren nach Anspruch 1, dadurch gekennzeichnet, daß Schritt c) folgende Teilschritte enthält:
α) Bildung von zu den Verlängerungsprodukten aus Schritt b) oder einem wesentlichen Teil davon komplementären Nukleinsäuren;
β) Verlängerung eines neuen Satzes der Oligonukleotide aus Schritt a) zu Polynukleotiden unter Verwendung der in α) gebildeten komplementären Nukleinsäuren als target-Nukleinsäuren;
γ) Nachweis der Verlängerungsprodukte aus α) oder β).

3. Verfahren gemäß Anspruch 1, dadurch gekennzeichnet, daß jeweils das X entsprechende Nukleotid eines Oligonukleotids zu dem Nukleotid X einer nachzuweisenden Nukleinsäure, nicht jedoch der anderen nachzuweisenden Nukleinsäuren, komplementär ist.

4. Verfahren nach Anspruch 3, dadurch gekennzeichnet, daß die Oligonukleotide mit dem Position X entsprechenden Nukleotid enden.

5. Verfahren nach Anspruch 1 oder 2, dadurch gekennzeichnet, daß die Positionen Y aus dem Bereich der Oligonukleotide gewählt werden, der 1 bis 8 Nukleotide vom endständigen Nukleotid der Oligonukleotide entfernt ist.

6. Verfahren nach einem der vorangehenden Ansprüche, dadurch gekennzeichnet, daß die Verlängerungsreaktionen der Oligonukleotide simultan erfolgen.

7. Verfahren nach einem der vorangehenden Ansprüche, dadurch gekennzeichnet, daß sich die Oligonukleotide detektierbar unterscheiden.

8. Verfahren nach einem der vorangehenden Ansprüche, dadurch gekennzeichnet, daß die Oligonukleotide unterschiedlich lang sind.

9. Verfahren nach einem der vorangehenden Ansprüche, dadurch gekennzeichnet, daß die Oligonukleotide unterschiedliche detektierbare Gruppen aufweisen.

10. Verfahren gemäß Anspruch 1, bei dem der Nachweis der Verlängerungsprodukte durch selektive Hybridisierung mit einer Sonde erfolgt.

11. Verfahren gemäß Anspruch 2, bei dem der Nachweis der komplementären Nukleinsäuren durch selektive Hybridisierung mit einer Sonde erfolgt.

12. Verfahren gemäß Anspruch 10 oder 11, dadurch gekennzeichnet, daß die Sonde einen ersten Teil besitzt, der im Bereich der Oligonukleotide anhybridisiert sowie einen zweiten Teil, der im Bereich der Verlängerung hybridisiert.

13. Verfahren gemäß Anspruch 1 oder 2, wobei der Nachweis der Verlängerungsprodukte darauf beruht, daß sich die Verlängerungsprodukte für verschiedene nachzuweisende Nukleinsäuren bevorzugt um mindestens drei Nukleotide voneinander unterscheiden.

14. Verfahren gemäß Anspruch 10 oder 11, bei dem der Nachweis der Verlängerungsprodukte zugleich eine Selektion auf das richtige Verlängerungsprodukt und auf die verschiedenen Allele liefert.

## Claims

1. Method for detecting similar nucleic acids whose nucleotide sequences differ from one another in at least one position (X) which comprises at least the following steps:
a) a set of oligonucleotides whose nucleotide sequences differ from one another in the position which corresponds to position X is reacted with the nucleic acids to be detected under hybridization conditions,
b) the hybridized oligonucleotides are extended in a single reaction vessel using the nucleic acids to be detected as a template and
c) the extension products are detected
wherein
- the oligonucleotides of the set additionally differ from one another in at least one additional position (Y), positions X and Y being located in the hybridizing region, such that the extension products from b) differ from one another in more than one position in the hybridizing region and
- in step c) the differences generated in step b) between the extension products and the similar nucleic acids and position (X) are used to detect the extension products.

2. Method as claimed in claim 1, wherein step c) comprises the following steps:
α) formation of nucleic acids complementary to the extension products from step b) or to a substantial part thereof;
β) extension of a new set of oligonucleotides from step a) to form polynucleotides using the complementary nucleic acids formed in α) as target nucleic acids;
γ) detection of the extension products from α) or β).

3. Method as claimed in claim 1, wherein in each case the nucleotide of an oligonucleotide that corresponds to X is complementary to the nucleotide X of one nucleic acid to be detected but not of the other nucleic acids to be detected.

4. Method as claimed in claim 3, wherein the oligonucleotides terminate with the nucleotide which corresponds to position X.

5. Method as claimed in claim 1 or 2, wherein the positions Y are selected from the oligonucleotide region which is located at a distance of 1 to 8 nucleotides from the terminal nucleotide of the oligonucleotides.

6. Method as claimed in one of the previous claims, wherein the extension reactions of the oligonucleotides occur simultaneously.

7. Method as claimed in one of the previous claims, wherein the oligonucleotides differ from one another in a detectable way.

8. Method as claimed in one of the previous claims, wherein the oligonucleotides differ in length.

9. Method as claimed in one of the previous claims, wherein the oligonucleotides have different detectable groups.

10. Method as claimed in claim 1, wherein the extension products are detected by selective hybridization with a probe.

11. Method as claimed in claim 2, wherein the complementary nucleic acids are detected by selective hybridization with a probe.

12. Method as claimed in claim 10 or 11, wherein the probe has a first part which hybridizes in the region of the oligonucleotides and a second part which hybridizes in the region of the extension.

13. Method as claimed in claim 1 or 2, wherein the detection of the extension products is based on the fact that the extension products for various nucleic acids to be detected differ from one another by preferably at least three nucleotides.

14. Method as claimed in claim 10 or 11, wherein the detection of the extension products at the same time provides a selection for the correct extension product and for the different alleles.

## Revendications

1. Procédé de détection d'acides nucléiques similaires, dont les séquences de nucléotides se distinguent à au moins une position (X), qui comprend au moins les étapes partielles suivantes :
(a) la réaction d'un jeu d'oligonucléotides, dont les séquences de nucléotides se distinguent à une position correspondant à la position (X), avec les acides nucléiques à détecter dans des conditions d'hybridation ;
(b) l'allongement des oligonucléotides hybridés en utilisant les acides nucléiques à détecter comme modèles dans un seul récipient de réaction ; et
(c) la détection des produits d'allongement ;
caractérisé en ce que
- les oligonucléotides du jeu se distinguent en outre à au moins une position supplémentaire (Y), les positions X et Y se trouvant dans la région d'hybridation, de telle manière que les produits d'allongement de l'étape b) se distinguent les uns des autres en plus d'une position dans la région d'hybridation et
- à l'étape c), on utilise les distinctions des produits d'allongement réalisées à l'étape b) par rapport aux acides nucléiques similaires, de même que la position (X) pour la détection des produits d'allongement.

2. Procédé selon la revendication 1, caractérisé en ce que l'étape c) comprend les étapes partielles suivantes :
α) la formation d'acides nucléiques complémentaires aux produits d'allongement de l'étape b) ou à une partie essentielle de ceux-ci ;
β) l'allongement d'un nouveau jeu d'oligonucléotides de l'étape a) en polynucléotides en utilisant les acides nucléiques complémentaires formés à l'étape α) à titre d'acides nucléiques cibles ;
γ) la détection des produits d'allongement de l'étape α) ou β).

3. Procédé selon la revendication 1, caractérisé en ce que le nucléotide correspondant à X d'un oligonucléotide est complémentaire au nucléotide X d'un acide nucléique à détecter, mais non aux autres acides nucléiques à détecter.

4. Procédé selon la revendication 3, caractérisé en ce que les oligonucléotides se terminent au nucléotide correspondant à la position X.

5. Procédé selon la revendication 1 ou 2, caractérisé en ce que les positions Y sont choisies dans la région des oligonucléotides qui est distante de 1 à 8 nucléotides du nucléotide terminal des oligonucléotides.

6. Procédé selon l'une des revendications précédentes caractérisé en ce que les réactions d'allongement des oligonucléotides sont effectuées simultanément.

7. Procédé selon l'une des revendications précédentes caractérisé en ce que les oligonucléotides se distinguent de façon détectable.

8. Procédé selon l'une des revendications précédentes caractérisé en ce que les oligonucléotides sont de longueur différente.

9. Procédé selon l'une des revendications précédentes caractérisé en ce que les oligonucléotides présentent des groupes détectables différents.

10. Procédé selon la revendication 1, dans lequel la détection des produits d'allongement est effectuée par hybridation sélective au moyen d'une sonde.

11. Procédé selon la revendication 2, dans lequel la détection des acides nucléiques complémentaires est effectuée par hybridation sélective au moyen d'une sonde.

12. Procédé selon la revendication 10 ou 11, caractérisé en ce que la sonde possède une première partie qui s'hybride dans la région des oligonucléotides, de même qu'une deuxième partie qui s'hybride dans la région de l'allongement.

13. Procédé selon la revendication 1 ou 2, dans lequel la détection des produits d'allongement est basée sur le fait que les produits d'allongement pour des acides nucléiques à détecter différents se distinguent de préférence par au moins trois nucléotides.

14. Procédé selon la revendication 10 ou 11, dans lequel la détection des produits d'allongement donne en même temps une sélection du produit d'allongement correct et des différents allèles.
